(19) Europäisches Patentamt
European Patent Office
Office européen des brevets

(11) EP 4 556 455 A1

(12) EUROPEAN PATENT APPLICATION
published in accordance with Art. 153(4) EPC

(43) Date of publication:
**21.05.2025 Bulletin 2025/21**

(21) Application number: **23838935.7**

(22) Date of filing: **11.07.2023**

(51) International Patent Classification (IPC):
***C07C 7/13*** *(2006.01)* ***C07C 15/08*** *(2006.01)*

(52) Cooperative Patent Classification (CPC):
**B01J 20/18; C07C 7/00; C07C 7/04; C07C 7/13; C07C 15/08**

(86) International application number:
**PCT/CN2023/106757**

(87) International publication number:
**WO 2024/012444 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.07.2022 CN 202210853808**

(71) Applicants:
- **China Petroleum & Chemical Corporation**
  **Beijing 100728 (CN)**
- **Sinopec Research Institute of Petroleum Processing Co., Ltd.**
  **Beijing 100083 (CN)**

(72) Inventors:
- **QIAO, Xiaofei**
  **Beijing 100083 (CN)**
- **MA, Jianfeng**
  **Beijing 100083 (CN)**
- **HUANG, Jian**
  **Beijing 100083 (CN)**
- **YANG, Yanqiang**
  **Beijing 100083 (CN)**
- **WANG, Hongchao**
  **Beijing 100083 (CN)**
- **LIU, Weiqiang**
  **Beijing 100083 (CN)**

(74) Representative: **Hoffmann Eitle**
**Patent- und Rechtsanwälte PartmbB**
**Arabellastraße 30**
**81925 München (DE)**

# (54) ALKYLBENZENE-CONTAINING DESORBENT AND USE THEREOF IN ADSORPTION AND SEPARATION OF META-AROMATIC HYDROCARBONS

(57) Disclosed are an alkylbenzene-containing desorbent and its use in the adsorption-separation of meta-aromatic, wherein the desorbent comprises or consists of 20-100 wt% of an alkylbenzene compound of the following general formula (I) and 0-80 wt% of a C5-C14 saturated aliphatic hydrocarbon, wherein $R_1$, $R_2$ and $R_3$ are independently selected from $C_{1-4}$ chain alkyl groups, and $R_4$, $R_5$ and R6 are independently selected from hydrogen, $C_{1-4}$ saturated hydrocarbon groups, $C_{1-4}$ alkoxy groups and halogens. The desorbent has a wide range of sources and an adsorption selectivity that is more similar to the target product, which is beneficial for improving yield and purity of product.

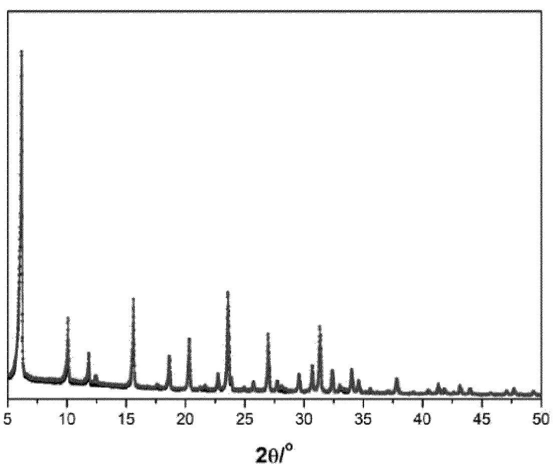


FIG. 1

EP 4 556 455 A1

## Description

### Technical Field

The present application relates to the separation and purification of meta-aromatic hydrocarbon(s), and in particular to a desorbent containing alkylbenzene and its use in the adsorption-separation of meta-aromatic hydrocarbon(s).

### Background Art

M-xylene (m-Xylene, MX) is an important basic organic chemical raw material, widely used in many fields such as synthetic resins, pesticides, medicines, coatings and dyes. Highly-pure M-xylene is usually obtained by separation of a mixed C8 aromatic hydrocarbons containing ethylbenzene, p-xylene, M-xylene and o-xylene. Since the boiling points of the four C8 aromatic isomers are similar, it is difficult to separate them by using conventional distillation processes; therefore, the processes for separating M-xylene mainly include sulfonation hydrolysis, complexation separation, extractive distillation and adsorption-separation. Wherein, the adsorption-separation method has the advantages of being environmentally friendly, pollution-free, corrosion-free, low equipment cost, high product purity and yield, and long adsorbent service life. It is the main development direction of m-xylene separation technology.

CN101745364A discloses an adsorbent for adsorbing and separating M-xylene and a preparation method thereof, wherein the adsorbent comprises Y zeolite exchanged with IA group metal ions and copper or silver ions and a binder, and toluene is used as a desorbent, with fast mass transfer speed and low usage amount.

CN101772478A discloses a method for separating M-xylene with a purity of at least 99 wt% by using simulated moving bed separation adsorption. The number of moving bed layers can be configured to be 12, 13 or 15, and the desorbent is toluene or a mixture of toluene and tetralin.

CN1939883A discloses a method for separating M-xylene by using faujasite-type zeolite adsorbents, which uses tetralin and its alkylated derivatives as desorbents, thereby reducing the cost of recovering and reusing the desorbents.

CN1379007A discloses a method for co-producing p-xylene and m-xylene including two-stage separation, which uses two separation steps, which use barium-exchanged X zeolite and potassium-exchanged Y zeolite as adsorbent components respectively, and uses p-diethylbenzene or p-difluorobenzene (first separation step) and toluene, indane or p-toluene as adsorbents to produce qualified paraxylene and metaxylene.

US5900523A discloses using sodium-exchanged Y zeolite as an active component of an adsorbent and indane as a desorbent can recover M-xylene in a single extraction raffinate without the need for an expensive fractionation process to remove o-xylene.

However, when toluene is used as a desorbent, its mass fraction in the extract and the raffinate is 80%-90%, and the heat load required for its separation is high, and the energy consumption is large. It is of limited sources, low content, and difficult to obtain, and when aromatic hydrocarbons (such as indane or tetralin) and their derivatives having a large difference in adsorption selectivity with the target product are used as desorbents for separation, the material and energy consumptions are large, the operating cost is high, and the yield of the target product is low.

Therefore, it is necessary to find a desorbent, which has a boiling point significantly different from that of the target product, a wide source, low cost, and can improve the separation effect of the target product, so as to effectively reduce production costs and improve the economic benefits of the device.

### Summary of The Invention

The object of the present application is to provide an alkylbenzene-containing desorbent and its use in the adsorption-separation of meta-aromatic hydrocarbon(s), wherein the desorbent can effectively achieve the desorption of the target product from the adsorbent, and its boiling point is greatly different from that of the target product, thereby facilitating subsequent separation from the target product by distillation for recycling and reuse.

In order to achieve the above object, in one aspect, the present application provides a use of a liquid material comprising or consisting of 20-100 wt% of an alkylbenzene compound of the following general formula (I) and 0-80 wt% of a C5-C14 saturated aliphatic hydrocarbon as a desorbent for the adsorption-separation of meta-aromatic hydrocarbon(s),

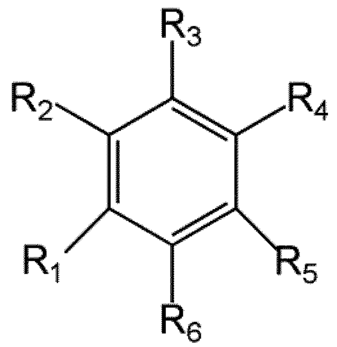

(I),

wherein:

- $R_1$, $R_2$ and $R_3$, which may be the same or different, are independently selected from $C_{1-4}$ alkyl groups;
- $R_4$, $R_5$ and $R_6$, which may be the same or different, are independently selected from hydrogen, $C_{1-4}$ saturated hydrocarbon group, $C_{1-4}$ alkoxy group and halogen.

In another aspect, the present application provides a method for separating meta-aromatic hydrocarbon(s) from a mixed aromatic hydrocarbon feedstock containing the meta-aromatic hydrocarbon(s) and isomers thereof, the method comprising the following steps:

1) the mixed aromatic hydrocarbon feedstock is contacted with an adsorbent to adsorb the meta-aromatic hydrocarbon(s), to obtain an adsorbent adsorbed with the meta-aromatic hydrocarbon(s) and a raffinate containing non-adsorbed components;
2) the adsorbent adsorbed with the meta-aromatic hydrocarbon(s) obtained in step 1) is contacted with a desorbent to desorb the meta-aromatic hydrocarbon(s), to obtain an extract liquid containing the meta-aromatic hydrocarbon(s) and the desorbent; and
3) the extract liquid obtained in step 2) is subjected to a rectification-separation to obtain the meta-aromatic hydrocarbon(s) and the desorbent,

wherein, based on the total amount of the desorbent, the desorbent comprises or consists of 20-100 wt% of the alkylbenzene compound of the above general formula (I) and 0-80 wt% of C5-C14 saturated aliphatic hydrocarbons.

In the third aspect, the present application provides an adsorption-desorption agents kit, comprising a solid adsorbent and a liquid desorbent, wherein the solid adsorbent contains at least 90 wt% of a Y-type molecular sieve as an active component, the Y-type molecular sieve has a silicon oxide/aluminum oxide molar ratio of 4.0-6.0, and the liquid desorbent contains or consists of 20-100 wt% of an alkylbenzene compound having the above general formula (I) and 0-80 wt% of a C5-C14 saturated aliphatic hydrocarbon.

The alkylbenzene-containing desorbent of the present application has a wide source; compared with the toluene desorbent, it has an adsorption selectivity which is similar to that of meta-aromatic hydrocarbon(s), which can effectively improve the yield of the target meta-aromatic product; and its boiling point is quite different from that of the target product, which is conducive to subsequent separation from the target product by distillation for recycling and reuse. For example, when the desorbent is used for the separation of m-xylene, the boiling point thereof is significantly higher than that of C8 aromatic hydrocarbons (for example, it can be more than 30 °C), which is conducive to its recycling and reuse after recovery from the kettle of the tower through distillation treatment, greatly reducing the energy consumption required for distillation of a large amount of desorbent with low boiling point to the top of the tower for recovery in devices currently using the desorbent with low boiling point such as toluene, effectively saving production costs. Utilizing the desorbent of the present application to separate meta-aromatic hydrocarbon(s) from the mixed aromatic hydrocarbon feedstock through adsorption-desorption separation has strong adaptability to the feedstock, and process parameters can be accurately controlled in the entire separation operation process to efficiently obtain the meta-aromatic products with high purity and yield; it is environmentally friendly and can significantly improve the economic benefits of the comprehensive utilization of C9+ heavy aromatics.

Other features and advantages of the present application will be described in detail in the subsequent specific embodiment section.

## Brief Description of Figures

The figures are used to provide a further understanding of the present application and constitute a part of the specification. Together with the following specific embodiments, the figures are used to explain the present application but do not constitute a limitation to the present application. In the figures:

FIG.1 is an X-ray diffraction spectrum of the molecular sieve of adsorbent preparation example 1;

FIG.2 is a pulse spectrum diagram of Example 1;

FIG.3 is a pulse spectrum diagram of Example 6;

FIG.4 is a pulse spectrum diagram of Example 9;

FIG.5 is a pulse spectrum diagram of Example 10;

FIG.6 is a pulse spectrum diagram of Comparative Example 1;

FIG.7 is a pulse spectrum diagram of Comparative Example 2;

FIG.8 is a pulse spectrum diagram of Comparative Example 3; and

FIG.9 is a schematic diagram of simulated moving bed adsorption-separation of the present application.

## Detailed Description of the Invention

The specific embodiments of the present application are described in detail below in conjunction with the figures. It should be understood that the specific embodiments described herein is only used to illustrate and explain the present application, and is not used to limit the present application.

The word "exemplary" used exclusively herein means "serving as an example, embodiment, or illustrative." Any example described herein as "exemplary" is not necessarily to be construed as being preferred or advantageous over other examples. Although various aspects of the examples are shown in the figures, the figures are not necessarily drawn to scale unless otherwise noted.

Any specific numerical value disclosed herein (including the endpoint of the numerical range) is not limited to the exact value of the numerical value, but should be understood to also cover values close to the exact value, such as all possible values within the range of $\pm5\%$ of the exact value. In addition, for the disclosed numerical range, the endpoint values of the range, the endpoint values and the specific point values in the range, and the specific point values can be arbitrarily combined to obtain one or more new numerical ranges, and these new numerical ranges should also be regarded as having been specifically disclosed herein.

Unless otherwise specified, the terms used herein have the same meaning as commonly understood by those skilled in the art. If a term is defined herein and its definition is different from the commonly understood meaning in the art, the definition herein shall prevail.

In the present application, the term "meta-aromatic hydrocarbon(s)" is also referred to as "meta-substituted aromatic hydrocarbon(s)", which refers to an aromatic compound which has only two substituents on the aromatic ring, wherein one of the substituents is in the relative meta position of the other substituent. According to the present application, the substituents on the meta-aromatic hydrocarbon do not contain Group VIA heteroatoms and Group VIIA heteroatoms, for example, it may include but is not limited to hydrocarbon groups, amine groups, etc., preferably hydrocarbon groups. Further preferably, the meta-aromatic hydrocarbon is a meta-alkyl aromatic hydrocarbon, that is, a meta-aromatic hydrocarbon in which both substituents are alkyl groups. Particularly preferably, the meta-aromatic hydrocarbon is a C8-C12 meta-aromatic hydrocarbon, more preferably a C8-C12 meta-alkyl aromatic hydrocarbon, such as m-xylene or 2,7-dimethylnaphthalene.

In the present application, the term "saturated aliphatic hydrocarbon" has the meaning generally understood in the art, including paraffin and cycloparaffin.

In the present application, the term "paraffin" has the meaning generally understood in the art, including normal paraffins (also called straight-chain alkanes) and isoparaffins (also called branched-chain alkanes).

In the present application, the term "saturated hydrocarbon group" has a meaning generally understood in the art, including chain alkyl groups and cycloalkyl groups.

In the present application, the term "alkyl group" has a meaning generally understood in the art, including straight-chain alkyl groups and branched-chain alkyl groups.

In the present application, the term "C8" refers to having 8 carbon atoms, and the term "C8 aromatic hydrocarbon" refers to an aromatic hydrocarbon having 8 carbon atoms.

In the present application, the term "C9" refers to having 9 carbon atoms, and the term "C9+ aromatic hydrocarbons" refers to aromatic hydrocarbons having more than 9 carbon atoms, also referred to herein as "heavy aromatic hydrocarbons".

In the present application, unless otherwise indicated, all pressures given are gauge pressures.

In the present application, except for the contents clearly stated, any matters or items not mentioned are directly applicable to those known in the art without any changes. Moreover, any embodiment described herein can be freely combined with one or more other embodiments described herein, and the technical solutions or technical ideas formed thereby are deemed to be part of the original disclosure or original record of this application, and should not be regarded as new contents not disclosed or anticipated herein, unless a person skilled in the art considers that the combination is obviously unreasonable.

All patent and non-patent literatures cited herein, including but not limited to textbooks and journal articles etc., are incorporated herein by reference in their entirety.

In the adsorption system, the metal ions in the molecular sieve crystals of the active component of the adsorbent are located above the aromatic ring of the meta-aromatic hydrocarbon and are biased towards the side of the aromatic ring with substituents, therefore, its area with a stronger positive electrostatic potential is biased towards the side of the aromatic ring, and the positive extreme value is located on one side of the aromatic ring. The two substituents of the meta-aromatic hydrocarbon are located on one side, and the angle formed is about 120 degrees. The negative electrostatic potential of the aromatic ring is distributed on one side of the aromatic ring, and the dispersion is good, and the negative extreme value site is relatively not centered, therefore, the area on one side of the aromatic ring is the position where the electrostatic attraction between the adsorbent and the adsorbate is the strongest. The adsorbent can preferentially adsorb meta-aromatic hydrocarbon(s) relative to other impurities. When other impurities have been removed from this system, the target component needs to be desorbed from the adsorbent to produce a highly-pure component for being processed and utilized. Whether efficient, high cycle rate, and high-purity desorption can be carried out at this time mainly depends on the properties of the desorbent, which needs to interact with the adsorbent to a certain extent, which needs to have a certain interaction with the adsorbent; this interaction force cannot be too strong or too weak; if it is too strong, the target component will be quickly desorbed and cannot be separated from other impurities, so that it cannot be effectively separated from other impurities, and the purity of the product cannot be guaranteed; if it is too weak, the desorption process is very slow, the consumption amount is very large, the timeliness and economy are too poor, and the target component is difficult to be desorbed, which will occupy the effective pore volume of the adsorbent and affect the next processing volume; at the same time, the negative electrostatic potential distribution area of the desorbent must match the positive electrostatic potential distribution area of the metal ion, the interaction area of the target component must match that of the impurity molecules; only when the interactions between the desorbent and the adsorbent and the target component are appropriate, can the target component be replaced from the adsorbent, and the adsorption and desorption process can be repeated quickly and efficiently to produce a highly-pure product. Therefore, finding a desorbent with a suitable negative electrostatic potential distribution area and suitable interaction with the adsorbent and the adsorbate can improve the adsorption-separation effect. In addition, in the adsorbent-desorbent system, there are intermolecular interactions between the target product and other impurity components, desorbent molecules, such as dispersion force, induction force, and mutual repulsion force, which will affect the diffusion coefficient of each component molecule within and between adsorbent crystals, thereby affecting the preferential adsorption selectivity of each component in this adsorption-desorption system. Therefore, different composition of feedstock and different adsorption-desorption systems will produce different adsorption effects.

As described above, in the first aspect, the present application provides a use of a liquid material comprising or consisting of 20-100 wt% of an alkylbenzene compound of the following general formula (I) and 0-80 wt% of a C5-C14 saturated aliphatic hydrocarbon as a desorbent for the adsorption-separation of meta-aromatic hydrocarbon(s),

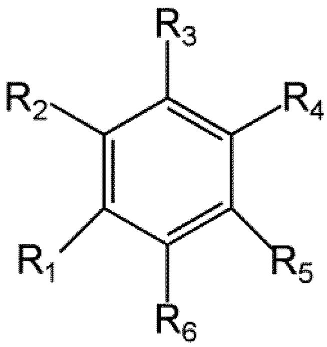

(I).

According to the present application, in the general formula (I), $R_1$, $R_2$ and $R_3$, which may be the same or different, are independently selected from $C_{1-4}$ alkyl groups, such as methyl, ethyl, propyl, butyl, including their various isomers; preferably at least one of $R_1$, $R_2$ and $R_3$ is methyl, more preferably at least two are methyl, and further preferably all are methyl.

According to the present application, in the general formula (I), $R_4$, $R_5$ and $R_6$, which may be the same or different, are independently selected from hydrogen, $C_{1-4}$ saturated hydrocarbon group, $C_{1-4}$ alkoxy group and halogen, wherein the $C_{1-4}$ saturated hydrocarbon group includes but is not limited to methyl, ethyl, propyl, butyl, isopropyl, isobutyl, tert-butyl, cyclopropyl, cyclobutyl, including various isomers thereof, preferably methyl; the $C_{1-4}$ alkoxy group includes but is not limited to methoxy, ethoxy, propoxy, butoxy, cyclopropyloxy, cyclobutyloxy, including various isomers thereof, and the halogen includes but is not limited to fluorine, chlorine and bromine; preferably, at least one of $R_4$, $R_5$ and $R_6$ is hydrogen or a $C_{1-4}$ saturated hydrocarbon group, for example, $R_4$ or $R_6$ is a $C_{1-4}$ saturated hydrocarbon group; more preferably, at least two of them are independently hydrogen or a $C_{1-4}$ saturated hydrocarbon group, for example, $R_4$ and $R_6$ are both $C_{1-4}$ saturated hydrocarbon groups; further preferably, all three are independently hydrogen or a $C_{1-4}$ saturated hydrocarbon group, and particularly preferably, all three are hydrogen.

In certain preferred embodiments, in formula (I), at least one of $R_1$, $R_2$ and $R_3$ is methyl, and $R_4$, $R_5$ and $R_6$ are independently hydrogen or a $C_{1-4}$ saturated hydrocarbon group.

In a further preferred embodiment, in formula (I), at least two of $R_1$, $R_2$ and $R_3$ are methyl, and $R_4$, $R_5$ and $R_6$ are independently hydrogen or a $C_{1-4}$ saturated hydrocarbon groups, and at least one of them is hydrogen.

In a further preferred embodiment, in formula (I), at least two of $R_1$, $R_2$ and $R_3$ are methyl, $R_5$ and $R_6$ are independently hydrogen or a $C_{1-4}$ saturated hydrocarbon group, and at least two of them are hydrogen, preferably all three are hydrogen.

According to the present application, the liquid material as a desorbent can be a C9+ heavy aromatic hydrocarbon having a specific structure, a C9+ heavy aromatic hydrocarbon derivative or a mixture thereof with an alkane. Preferably, the C9+ heavy aromatic hydrocarbon is selected from one or more of the following: 1,2,3-trisubstituted C9+ alkylbenzene or its derivative, 1,2,3,4-tetrasubstituted C9+ alkylbenzene or its derivative, 1,2,3,4,5-pentasubstituted C9+ alkylbenzene or its derivative. In a particularly preferred embodiment, the alkylbenzene compound of formula (I) is selected from 1,2,3-trimethylbenzene (also referred to as tritoluene herein), 1,2,3,4-tetramethylbenzene (also referred to as tetratoluene herein), 1,2,3,4,5-pentamethylbenzene (also referred to as pentatoluene herein) and 3-ethyl-o-xylene, more preferably 1,2,3-trimethylbenzene or 1,2,3,4-tetramethylbenzene.

In recent years, with the gradual commissioning of large-scale integrated naphtha reforming, ethylene and aromatics combined units in our country, the output of heavy aromatics has also been increasing. Wherein, the mass fraction of C9+ heavy aromatics in the catalytic reforming reaction products can usually reach 20%-50%, and the content of heteroatom components and olefins is low and the stability is good, so it is a high-quality raw material for further processing and utilization. The mass fraction of trimethylbenzene in the reformed C9+ heavy aromatics is 15%-30%, and the mass fraction of tetramethylbenzene is 3%-8%. At present, the pseudocumene and mesitylene in this part of trimethylbenzene and tetramethylbenzene have been separated through mature industrial production processes and used to produce partial anhydride and trimesic acid, while the utilization of tritoluene and tetratoluene is relatively weak, and most of them are sold as high-boiling point aromatic solvent oil with low added value. The present application utilizes C9+ heavy aromatic hydrocarbons, such as 1,2,3-trimethylbenzene, as a desorbent for separating meta-aromatic hydrocarbon(s); this not only achieves high-value utilization of C9+ heavy aromatic hydrocarbon resources, but also helps to reduce energy and material consumptions of hydrocarbon adsorption-separation devices of meta aromatic; it not only brings considerable economic benefits to production enterprises, but also benefits energy conservation and consumption reduction for enterprises.

In a preferred embodiment, the adsorption-separation comprises separating a meta-aromatic hydrocarbon from a mixed aromatic hydrocarbon feedstock comprising the meta-aromatic hydrocarbon and at least one isomer thereof by

adsorption and desorption.

In a further preferred embodiment, the meta-aromatic hydrocarbon is a C8-C12 meta-aromatic hydrocarbon, more preferably a C8-C12 meta-alkyl aromatic hydrocarbon, such as m-xylene or 2,7-dimethylnaphthalene.

In certain further preferred embodiments, the meta-aromatic hydrocarbon is m-xylene, and the mixed aromatic hydrocarbon feedstock is a mixed C8 aromatic hydrocarbon feedstock comprising m-xylene and at least one other C8 aromatic hydrocarbon selected from p-xylene, o-xylene and ethylbenzene. Further preferably, the mixed C8 aromatic hydrocarbon feedstock comprises 5-95 wt% of m-xylene. Particularly preferably, the mixed aromatic hydrocarbon feedstock comprises 5-94 wt% of m-xylene and 6-95 wt% of p-xylene, more preferably 5-90 wt% of m-xylene and 10-95 wt% of p-xylene. For example, the content of xylene in the mixed aromatic hydrocarbon feedstock may be 10-90 wt%, 20-80 wt%, 30-70 wt% or 40-60 wt%; the content of p-xylene in the mixed aromatic hydrocarbon feedstock may be 6-50 wt%, 7-40 wt%, 8-30 wt% or 10-25 wt%.

In certain particularly preferred embodiments, the meta-aromatic hydrocarbon is m-xylene, and the mixed aromatic hydrocarbon feedstock comprises 20-60 wt% of m-xylene, 10-30 wt% of p-xylene, 10-30 wt% of o-xylene and 5-20 wt% of ethylbenzene, for example, comprises 40-55 wt% of m-xylene, 15-25 wt% of p-xylene, 15-25 wt% of o-xylene and 5-15 wt% of ethylbenzene.

In some further preferred embodiments, the meta-aromatic hydrocarbon is 2,7-dimethylnaphthalene, and the mixed aromatic hydrocarbon feedstock is a mixed C12 aromatic hydrocarbon feedstock comprising 2,7-dimethylnaphthalene (meta-position) and at least one other C12 aromatic hydrocarbon selected from 1,6-dimethylnaphthalene (non-para-, non-ortho-position), 2,6-dimethylnaphthalene (para-position) and 1,8-dimethylnaphthalene (ortho-position), 1,2-dimethylnaphthalene, 1,3-dimethylnaphthalene, 1,4-dimethylnaphthalene, 1,5-dimethylnaphthalene, 1,7-dimethylnaphthalene, 2,3 -dimethylnaphthalene. Further preferably, the mixed C12 aromatic hydrocarbon feedstock comprises 5-95 wt% of 2,7-dimethylnaphthalene.

In a preferred embodiment, the adsorbent used in the adsorption-separation comprises at least 90 wt% of a Y-type molecular sieve as an active component, and the Y-type molecular sieve has a silicon oxide/aluminum oxide molar ratio of 4.0-6.0, preferably 4.3-5.7. Other features of the adsorbent are as described in the second aspect of the application below.

In a preferred embodiment, based on the total amount of the liquid material, the liquid material comprises or consists of 30-95 wt%, preferably 30-80 wt%, more preferably 30-50 wt% of the alkylbenzene compound, and 5-70 wt%, preferably 20-70 wt%, more preferably 50-70 wt% of an alkane selected from C5-C14 normal alkanes, preferably selected from C6-C10 normal alkanes, more preferably selected from C6-C8 normal alkanes. The inventors of the present application have found that including a certain amount of C5-C14 normal alkanes in the liquid material as desorbent is conducive to alleviating the strong desorption performance of the aromatic desorbent, and is conducive to reducing the half-peak width of the separation peak, thereby improving the separation degree between the target component and other components, reducing the total amount of the required desorbent, and reducing material consumption and energy consumption.

In a particularly preferred embodiment, the meta-aromatic hydrocarbon is m-xylene, the mixed aromatic hydrocarbon feedstock comprises 20-60 wt% of m-xylene, 10-30 wt% of p-xylene, 10-30 wt% of o-xylene and 5-20 wt% of ethylbenzene, the alkylbenzene compound of formula (I) is selected from 1,2,3-trimethylbenzene, 1,2,3,4-tetramethylbenzene, 1,2,3,4,5-pentamethylbenzene and 3-ethyl-o-xylene; based on the total amount of the liquid material, the liquid material comprises 30-70 wt% of the alkylbenzene compound, and 30-70 wt% of alkanes selected from C5-C14 normal alkanes; and the charge-balancing cation of the adsorbent is selected from $Na^+$, $Sr^{2+}$, $Ba^{2+}$ and $Ag^+$, and the adsorbent contains 0.05-0.8 wt%, preferably 0.1-0.5 wt% of adsorbed water.

In the second aspect, the present application provides a method for separating meta-aromatic hydrocarbon(s) from a mixed aromatic hydrocarbon feedstock containing the meta-aromatic hydrocarbon(s) and their isomers, comprising the following steps:

1) the mixed aromatic hydrocarbon feedstock is contacted with an adsorbent to adsorb meta-aromatic hydrocarbon(s), thereby obtaining an adsorbent adsorbed with the meta-aromatic hydrocarbon(s) and a raffinate containing non-adsorbed components;
2) the adsorbent adsorbed with the meta-aromatic hydrocarbon(s) obtained in step 1) is contacted with a desorbent to desorb the meta-aromatic hydrocarbon(s), thereby obtaining an extract liquid containing the meta-aromatic hydrocarbon(s) and the desorbent; and
3) the extract liquid obtained in step 2) is subjected to a rectification-separation to obtain the meta-aromatic hydrocarbon(s) and the desorbent, wherein, based on the total amount of the desorbent, the desorbent comprises or consists of 20-100 wt% of an alkylbenzene compound of the following general formula (I) and 0-80 wt% of a C5-C14 saturated aliphatic hydrocarbon,

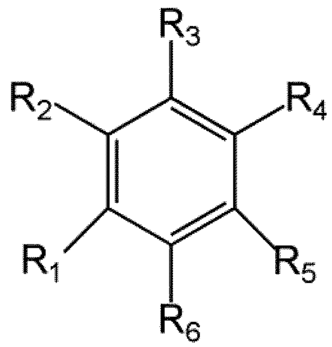

(I),

wherein $R_1$, $R_2$, $R_3$, $R_4$, $R_5$ and $R_6$ are as defined above.

In certain preferred embodiments, in formula (I), at least one of $R_1$, $R_2$ and $R_3$ is methyl, and $R_4$, $R_5$ and $R_6$ are independently hydrogen or a $C_{1-4}$ saturated hydrocarbon group.

In a further preferred embodiment, in formula (I), at least two of $R_1$, $R_2$ and $R_3$ are methyl, and $R_4$, $R_5$ and $R_6$ are independently hydrogen or a $C_{1-4}$ saturated hydrocarbon group, and at least one of them is hydrogen.

In a further preferred embodiment, in formula (I), at least two of $R_1$, $R_2$ and $R_3$ are methyl, $R_5$ and $R_6$ are independently hydrogen or a $C_{1-4}$ saturated hydrocarbon group, and at least two of them are hydrogen, preferably all three are hydrogen.

In a particularly preferred embodiment, the alkylbenzene compound of formula (I) is selected from 1,2,3-trimethylbenzene, 1,2,3,4-tetramethylbenzene, 1,2,3,4,5-pentamethylbenzene and 3-ethyl-o-xylene, preferably 1,2,3-trimethylbenzene or 1,2,3,4-tetramethylbenzene.

In a preferred embodiment, the meta-aromatic hydrocarbon is a C8-C12 meta-aromatic hydrocarbon, preferably a C8-C12 meta-alkyl aromatic hydrocarbon, such as m-xylene or 2,7-dimethylnaphthalene.

In certain further preferred embodiments, the meta-aromatic hydrocarbon is m-xylene, and the mixed aromatic hydrocarbon feedstock is a mixed C8 aromatic hydrocarbon feedstock comprising m-xylene and at least one other C8 aromatic hydrocarbon selected from p-xylene, o-xylene and ethylbenzene. Further preferably, the mixed C8 aromatic hydrocarbon feedstock comprises 5-95 wt% of m-xylene. Particularly preferably, the mixed aromatic hydrocarbon feedstock comprises 5-94 wt% of m-xylene and 6-95 wt% of p-xylene, more preferably 5-90 wt% of m-xylene and 10-95 wt% of p-xylene. For example, the content of xylene in the mixed aromatic hydrocarbon feedstock may be 10-90 wt%, 20-80 wt%, 30-70 wt% or 40-60 wt%; the content of p-xylene in the mixed aromatic hydrocarbon feedstock may be 6-50 wt%, 7-40 wt%, 8-30 wt% or 10-25 wt%.

In some further preferred embodiments, the meta-aromatic hydrocarbon is 2,7-dimethylnaphthalene, and the mixed aromatic hydrocarbon feedstock is a mixed C12 aromatic hydrocarbon feedstock comprising 2,7-dimethylnaphthalene (meta-position) and at least one other C12 aromatic hydrocarbon selected from 1,6 -dimethylnaphthalene (non-para-, non-ortho-position), 2,6-dimethylnaphthalene (para-position) and 1,8-dimethylnaphthalene (ortho-position). Further preferably, the mixed C12 aromatic hydrocarbon feedstock comprises 5-95 wt% of 2,7-dimethylnaphthalene.

The present application has no strict restrictions on the source of the mixed aromatic hydrocarbon feedstock. In certain exemplary embodiments, the mixed aromatic hydrocarbon feedstock, especially the mixed C8 aromatic hydrocarbon feedstock, may come from a catalytic reforming unit, a disproportionation and transalkylation unit, a toluene shape selective disproportionation unit, an isomerization unit and/or a p-xylene adsorption-separation unit.

In a preferred embodiment, based on the total amount of the desorbent, the desorbent comprises or consists of 30-95 wt%, preferably 30-80 wt%, more preferably 30-50 wt% of the alkylbenzene compound, and 5-70 wt%, preferably 20-70 wt%, more preferably 50-70 wt% of alkanes selected from C5-C14 normal alkanes, preferably selected from C6-C10 normal alkanes, more preferably selected from C6-C8 normal alkanes.

In a preferred embodiment, the adsorbent used in step 1) contains at least 90 wt% of Y-type molecular sieve as an active component, and the silicon oxide/aluminum oxide molar ratio of the Y-type molecular sieve is 4.0-6.0, preferably 4.3-5.7. Further preferably, the crystal size of the Y-type molecular sieve is 0.5-2.0 μm, preferably 0.8-1.2 μm. In certain exemplary embodiments, the toluene adsorption capacity of the finished beads of the adsorbent is 155-220 mg/g, the calcined-base bulk density is 0.645-0.867 g/mL, and the crushing rate at 130N is 0.3-5.0 wt%.

In a further preferred embodiment, the adsorbent contains 0.05-2 wt%, preferably 0.05-1 wt%, more preferably 0.05-0.8 wt%, and further preferably 0.1-0.5 wt% of adsorbed water. The inventors of the present application have found that the water molecules in the adsorbent have the effect of regulating the adsorption capacity and adsorption performance. When the water content in the adsorbent is different, the preferential selectivity of the adsorbent to the target component is different. Due to the polarization of water molecules, the polarization intensity of the cations outside the crystal framework of the adsorbent molecular sieve will be affected by water molecules, the interaction between the

adsorbent and the adsorbate, especially the electrostatic interaction between the adsorbent and the target component will change, so the selectivity of the adsorbent and the mass transfer efficiency of the adsorbate in the adsorption system will change. In addition, if the mass fraction of water molecules is high, a large number of water molecules occupy a part of the effective pore volume in the crystal framework of the adsorbent molecular sieve, so that the adsorption capacity of the target component is reduced; when the water content exceeds a certain range, the molecular sieve crystal structure of the adsorbent will undergo irreversible hydrothermal destruction, resulting in a significant reduction in adsorption capacity and adsorption selectivity. Therefore, the appropriate water content in the adsorbent is conducive to the adsorbent to stably exert the optimal adsorption performance for a long time.

The present application has no strict requirements for the preparation method of the adsorbent. In certain exemplary embodiments, the adsorbent is prepared by rolling ball mixing method, and the composition of the raw materials is 90-99 wt% of Y-type molecular sieve, 0.5-9 wt% of binder and 0.5-1 wt% of molding aid. Preferably, the binder is kaolin, bentonite and/or attapulgite, and the molding aid is lignin, sesbania powder, dry starch, carboxymethyl cellulose and/or activated carbon. After its preparation, the composition of the adsorbent is 91-99.5 wt% of Y-type molecular sieve and 0.5-9 wt% of binder.

In a further preferred embodiment, the adsorbent further comprises one or more, for example one or two, of groups IA, IIA and IB metal ions as charge-balancing cations, wherein the group IA metal ions are preferably selected from $Li^+$ and $Na^+$, the group IIA metal ions are preferably selected from $Mg^{2+}$, $Sr^{2+}$ and $Ba^{2+}$, and the group IB metal ions are preferably $Ag^+$. Preferably, the total amount of groups IA, IIA and IB metals in the adsorbent is 10-45% based on the total weight of the adsorbent and calculated as metal oxide.

In a particularly preferred embodiment, the adsorbent comprises at least 90 wt% of NaY-type molecular sieve as an active component, and the adsorbent is preferably treated with metal ion exchange, i.e., $Na^+$ ions in the molecular sieve are exchanged with other metal ions, so that the exchange degree of metal ions is 78.0-99.9%. Particularly preferably, the molar concentration of the metal salt in the ion exchange process is 0.05-0.65 mol/L, preferably 0.15-0.50 mol/L.

All features of the adsorbent disclosed in the second aspect of the present application are also applicable to the adsorbents described in the first and third aspects of the present application, and therefore they will not be described in detail in the first and third aspects of the present application.

In a particularly preferred embodiment, the meta-aromatic hydrocarbon is m-xylene, the mixed aromatic hydrocarbon feedstock comprises 20-60 wt% of m-xylene, 10-30 wt% of p-xylene, 10-30 wt% of o-xylene and 5-20 wt% of ethylbenzene, the alkylbenzene compound of formula (I) is selected from 1,2,3-trimethylbenzene, 1,2,3,4-tetramethylbenzene, 1,2,3,4,5-pentamethylbenzene and 3-ethyl-o-xylene; based on the total amount of the desorbent, the desorbent comprises 30-70 wt% of the alkylbenzene compound, and 30-70 wt% of an alkane selected from C5-C14 normal alkanes; and the charge-balancing cation of the adsorbent is selected from $Na^+$, $Sr^{2+}$, $Ba^{2+}$ and $Ag^+$, and the adsorbent contains 0.05-0.8 wt%, preferably 0.1-0.5 wt% of adsorbed water.

In a preferred embodiment, the operating conditions of the adsorption in step 1) and the desorption in step 2) each independently include:

a temperature of 100-190°C, preferably 110-180°C, more preferably 120-160°C; and/or
a pressure of 0.6-1.6 MPa, preferably 0.8-1.0 MPa.

According to the present application, the method for separating meta-aromatic hydrocarbon(s) can be implemented by using, but not limited to, a simulated moving bed. Generally, the simulated moving bed may include one or more adsorption towers, each tower containing a plurality of bed layers filled with adsorbents, each bed layer having its own material inlet and outlet pipelines, the material in the adsorption tower flows from top to bottom, the material between towers is transported by a circulating pump, and the material flows through the adsorption bed layers of different adsorption towers to form a closed cycle with end-to-end connections. The materials entering and exiting the adsorption bed layer at least include feedstock (F), desorbent (D), extract (E) and raffinate (R). The materials entering and exiting the simulated moving bed divide the adsorption bed layers therein into a desorption zone, a purification zone, an adsorption zone and an isolation zone, wherein the adsorption bed layers between the desorbent injection and the extract discharging are the desorption zone; the adsorption bed layers between the extract discharging and the feedstock injection are the purification zone; the adsorption bed layers between the feedstock injection and the raffinate discharging are the adsorption zone, and the temperature of adsorption bed layer is the adsorption temperature; and the adsorption bed layers between the raffinate discharging and the desorbent injection are the isolation zone. During the operation of the simulated moving bed, the positions of each stream of material entering and exiting the adsorption bed layers of the adsorption tower changes periodically. A multi-way rotary valve or a program-controlled switch valve group can be used to control the entry and exit of each stream of material in different adsorption bed layers. At a certain moment, each stream of material is connected to a specific bed layer, and at a certain interval, i.e., one step time, the entry and exit positions of each stream of material move down one adsorption bed layer. The time required for the entry position of a stream of material into the adsorption bed layer (or the discharging position of a stream of material from the adsorption bed) return to the starting position through all

adsorption bed layers is one cycle period.

In certain preferred embodiments, the steps 1) and 2) are performed by using a simulated moving bed, wherein the simulated moving bed comprises a plurality of adsorption bed layers filled with adsorbents, each bed layer being provided with a corresponding material inlet and outlet pipeline, and the materials entering and exiting the simulated moving bed divide the adsorption bed into a desorption zone, a purification zone, an adsorption zone and an isolation zone. Preferably, the ratio of the number of bed layers in the adsorption zone, the purification zone, the desorption zone and the isolation zone in the simulated moving bed is 25±10%: 38±15%: 25±5%: 12±4%.

In a further preferred embodiment, the mass flow rate ratio of the desorbent entering the simulated moving bed to the mixed aromatic hydrocarbon feedstock is 0.01-6.0, preferably 0.01-5.5, more preferably 0.01-5.0.

In a further preferred embodiment, relative to the unit mass of adsorbent, the flow rate of the mixed aromatic hydrocarbon feedstock entering the simulated moving bed is in the range of 0.1-8 kg/(h·kg adsorbent), preferably 0.15-8 kg/(h·kg adsorbent), and more preferably 0.17-8 kg/(h·kg adsorbent).

In a further preferred embodiment, the step time of the simulated moving bed is 60-160 seconds, preferably 70-120 seconds.

In a further preferred embodiment, one cycle period of the simulated moving bed is 12-70 minutes, preferably 20-40 minutes.

In the method of the present application, by selecting a suitable desorbent and corresponding operating conditions, when the separation of step 1) and step 2) is carried out by using a simulated moving bed, the separation effect can be improved, including purity and yield, the feedstock processing capacity is increased, and material consumption and energy consumption are reduced, including the pipeline flow rate and circulation flow rate of each stream of materials, as well as the energy consumption for recycling and reuse.

According to the present application, the extract liquid obtained in step 2) contains meta-aromatic hydrocarbon(s) and part of the desorbent, and the difference in boiling points between the two components can be used to separate and recover the two components through the distillation in step 3). For example, when the meta-aromatic hydrocarbon(s) is m-xylene, in step 3), the desorbent in the extract can be recovered by a distillation tower in the kettle of the tower, and the top stream passes through a subsequent product tower to obtain a highly-pure M-xylene product. In addition, when a simulated moving bed is used, the main components of the raffinate obtained in step 1) are the desorbent and other components in the feedstock except M-xylene. The desorbent in the raffinate can be recovered by a distillation tower in the kettle of the tower, and the raffinate oil obtained at the top of the tower can be subjected to corresponding subsequent processing and separation, or used as an isomerization feedstock for an aromatics unit.

In a third aspect, the present application provides an adsorption-desorption agents kit, comprising a solid adsorbent and a liquid desorbent, wherein the solid adsorbent comprises at least 90 wt% of a Y-type molecular sieve as an active component, the Y-type molecular sieve has a silicon oxide/aluminum oxide molar ratio of 4.0-6.0, preferably 4.3-5.7, and the liquid desorbent comprises or consists of 20-100 wt% of an alkylbenzene compound of the following general formula (I) and 0-80 wt% of a C5-C14 saturated aliphatic hydrocarbon,

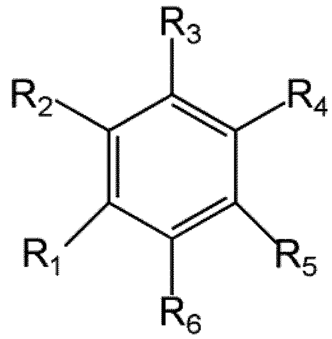

(I),

wherein $R_1$, $R_2$, $R_3$, $R_4$, $R_5$ and $R_6$ are as defined above.

In certain preferred embodiments, in formula (I), at least one of $R_1$, $R_2$ and $R_3$ is methyl, and $R_4$, $R_5$ and $R_6$ are independently hydrogen or a $C_{1-4}$ saturated hydrocarbon group.

In a further preferred embodiment, in formula (I), at least two of $R_1$, $R_2$ and $R_3$ are methyl, and $R_4$, $R_5$ and $R_6$ are independently hydrogen or a $C_{1-4}$ saturated hydrocarbon group, and at least one of them is hydrogen.

In a further preferred embodiment, in formula (I), at least two of $R_1$, $R_2$ and $R_3$ are methyl, $R_5$ and $R_6$ are independently hydrogen or a $C_{1-4}$ saturated hydrocarbon group, and at least two of them are hydrogen, preferably all three are hydrogen.

In a further preferred embodiment, the alkylbenzene compound of formula (I) is selected from 1,2,3-trimethylbenzene, 1,2,3,4-tetramethylbenzene, 1,2,3,4,5-pentamethylbenzene and 3-ethyl-o-xylene, preferably 1,2,3-trimethylbenzene or 1,2,3,4-tetramethylbenzene.

In a preferred embodiment, based on the total amount of the liquid desorbent, the desorbent comprises or consists of 30-95 wt%, preferably 30-80 wt%, more preferably 30-50 wt% of the alkylbenzene compound, and 5-70 wt%, preferably 20-70 wt%, more preferably 50-70 wt% of alkanes selected from C5-C14 normal alkanes, preferably selected from C6-C10 normal alkanes, more preferably selected from C6-C8 normal alkanes.

In a preferred embodiment, the solid adsorbent contains 0.05-2 wt%, preferably 0.05-1 wt%, more preferably 0.05-0.8 wt%, further preferably 0.1-0.5 wt% of adsorbed water.

In a particularly preferred embodiment, based on the total amount of the liquid desorbent, the liquid desorbent comprises 30-70 wt% of the alkylbenzene compound and 30-70 wt% of alkanes selected from C5-C14 normal alkanes; and the charge-balancing cations of the solid adsorbent are selected from $Na^+$, $Sr^{2+}$, $Ba^{2+}$ and $Ag^+$, and the adsorbent contains 0.05-0.8 wt%, preferably 0.1-0.5 wt% of adsorbed water.

Other features of the adsorbent used in the third aspect of the present application are as described in the second aspect of the present application and will not be repeated here.

In certain preferred embodiments, the present application provides the following technical solutions:

1. A method for separating highly-pure M-xylene by adsorption using a heavy desorbent, comprising: introducing a C8 aromatic hydrocarbon mixed feedstock into an adsorbent, wherein the M-xylene in the feedstock is adsorbed by the adsorbent, and the components not adsorbed are discharged as a raffinate, and a heavy desorbent is introduced into the adsorbent to desorb the adsorbed M-xylene to obtain an extract, and the extract and raffinate containing the desorbent are subjected to distillation separation to respectively obtain M-xylene and other C8 aromatic hydrocarbon components at the top of distillation towers, and the desorbent is recovered in the kettle of the distillation tower.
2. The method as described in item 1, characterized in that the heavy desorbent is one or more selected from the following: 1,2,3-trisubstituted C9+ alkylbenzene or its derivatives, 1,2,3,4-tetrasubstituted C9+ alkylbenzene or its derivatives, 1,2,3,4,5-pentasubstituted C9+ alkylbenzene or its derivatives.
3. The method as described in item 1, characterized in that the heavy desorbent is a mixture of alkanes and one or more selected from the following: 1,2,3-trisubstituted C9+ alkylbenzene or its derivatives, 1,2,3,4-tetrasubstituted C9+ alkylbenzene or its derivatives, 1,2,3,4,5-pentasubstituted C9+ alkylbenzene or its derivatives; wherein the alkanes are preferably C5-C8 normal alkanes, and the content thereof is not higher than 80 wt%.
4. The method as described in item 2 or 3, characterized in that the 1,2,3-trisubstituted C9+ alkylbenzene is 1,2,3-trimethylbenzene and 3-ethyl-o-xylene, the 1,2,3,4-tetrasubstituted C9+ alkylbenzene is 1,2,3,4-tetramethylbenzene, and the 1,2,3,4,5-pentasubstituted C9+ alkylbenzene is 1,2,3,4,5-pentamethylbenzene.
5. The method as described in any one of items 1 to 4, characterized in that the active component of the adsorbent is a Y-type molecular sieve, and its silicon oxide/aluminum oxide molar ratio is 4.0-6.0, preferably 4.3-5.7.
6. The method as described in item 5, characterized in that the crystal size of the Y-type molecular sieve is 0.5-2.0 $\mu$m, preferably 0.8-1.2 $\mu$m.
7. The method as described in any one of items 1 to 4, characterized in that the adsorbent contains no more than 2 wt%, preferably no more than 1 wt%, and more preferably no more than 0.8 wt% of adsorbed water.
8. The method as described in items 1 to 4, characterized in that the active component of the adsorbent is a Y-type molecular sieve, and the charge-balancing cations of the adsorbent are one or two metal ions of groups IA, IIA and IB, preferably, the metal ions in group IA are one or two of $Li^+$ and $Na^+$, the metal ions in group IIA are one or two of $Mg^{2+}$, $Sr^{2+}$ and $Ba^{2+}$, and the metal ion in group IB is $Ag^+$.
9. The method as described in items 1 to 4, characterized in that the adsorbent whose active component is NaY-type molecular sieve is subjected to a metal ion exchange, and the molar concentration of the metal salt during the ion exchange process is 0.05-0.65 mol/L, preferably 0.15-0.50 mol/L.
10. The method as described in any one of items 1 to 9, characterized in that the adsorption temperature is 100-190 °C, preferably 110-180 °C, and more preferably 120-160 °C.
11. The method as described in any one of items 1 to 10, characterized in that the adsorption pressure is 0.6-1.6 MPa, preferably 0.8-1.0 MPa.
12. The method as described in any one of items 1 to 11, characterized in that the feedstock comes from a catalytic reforming unit, a disproportionation and transalkylation unit, a toluene shape-selective disproportionation unit, an isomerization unit and/or a p-xylene adsorption-separation unit.
13. The method as described in any one of items 1 to 12, characterized in that the composition of the rolling-ball mixture used in the preparation process of adsorbent is: 90-99 wt% of Y-type molecular sieve, 0.5-9 wt% of binder and 0.5-1 wt% of molding aid, and the composition of the adsorbent is: 91.0-99.5 wt% of Y-type molecular sieve and 0.5-9 wt% of binder.
14. The method as described in item 13, characterized in that the binder is kaolin, bentonite and/or attapulgite.
15. The method as described in item 13, characterized in that the molding aid is lignin, sesbania powder, dry starch,

carboxymethyl cellulose and/or activated carbon.

16. The method as described in any one of items 1 to 15, characterized in that the adsorption-separation adopts a simulated moving bed process.

17. The method as described in item 16, characterized in that the simulated moving bed comprises a plurality of adsorption bed layers filled with adsorbents, each bed layer having its own material inlet and outlet pipelines, and the materials entering and exiting the simulated moving bed divide the adsorption bed layers therein into a desorption zone, a purification zone, an adsorption zone and an isolation zone, the adsorption bed layers between the desorbent injection and the extract discharging are the desorption zone, the adsorption bed layers between the extract discharging and the feedstock injection are the purification zone, the adsorption bed layers between the feedstock injection and the raffinate discharging are the adsorption zone, and the adsorption bed layers between the raffinate discharging and the desorbent injection are the isolation zone.

18. The method described in Item 16, characterized in that the ratio of the number of bed layer in the adsorption zone, purification zone, desorption zone and isolation zone in the simulated moving bed is 25±10%: 38±15%: 25±5%: 12 ±4%.

19. The method as described in item 16, characterized in that the mass flow rate ratio of the desorbent to the feedstock entering the simulated moving bed is not greater than 6.0, preferably not greater than 5.5, and more preferably not greater than 5.0.

20. The method as described in item 16, characterized in that the flow rate of feedstock entering the simulated moving bed relative to the unit mass of adsorbent is not less than 0.1 kg/(h·kg adsorbent), preferably not less than 0.15 kg/(h·kg adsorbent), and more preferably not less than 0.17 kg/(h·kg adsorbent).

21. The method as described in item 16, characterized in that one cycle period of the simulated moving bed is 12-70 minutes, preferably 20-40 minutes.

22. A desorbent composition comprising an alkylbenzene selected from the following and one or more alkanes selected from C5-C8 normal alkanes, wherein the alkylbenzene is one or more selected from the following: 1,2,3-trisubstituted C9+ alkylbenzene or its derivatives, 1,2,3,4-tetrasubstituted C9+ alkylbenzene or its derivatives, 1,2,3,4,5-pentasubstituted C9+ alkylbenzene or its derivatives, and the content of the alkane is not higher than 80wt% based on the weight of the desorbent composition.

23. The composition as described in item 22, characterized in that the 1,2,3-trisubstituted C9+ alkylbenzene is 1,2,3-trimethylbenzene and 3-ethyl-o-xylene, the 1,2,3,4-tetrasubstituted C9+ alkylbenzene is 1,2,3,4-tetramethylbenzene, and the 1,2,3,4,5-pentasubstituted C9+ alkylbenzene is 1,2,3,4,5-pentamethylbenzene.

24. Use of an alkylbenzene selected from the following as a desorbent for the adsorption-separation of highly-pure M-xylene, wherein the alkylbenzene is one or more selected from the following: 1,2,3-trisubstituted C9+ alkylbenzene or its derivatives, 1,2,3,4-tetrasubstituted C9+ alkylbenzene or its derivatives, 1,2,3,4,5-pentasubstituted C9+ alkylbenzene or its derivatives, and the content of the alkane is not higher than 80wt% based on the weight of the desorbent composition.

25. The use as described in item 22, wherein the alkylbenzene is combined with one or more alkanes selected from C5-C8 normal alkanes as the desorbent, wherein the content of the alkane is not higher than 80wt% based on the weight of the desorbent.

26. The use as described in item 24 or 25, characterized in that the 1,2,3-trisubstituted C9+ alkylbenzene is 1,2,3-trimethylbenzene and 3-ethyl-o-xylene, the 1,2,3,4-tetrasubstituted C9+ alkylbenzene is 1,2,3,4-tetramethylbenzene, and the 1,2,3,4,5-pentasubstituted C9+ alkylbenzene is 1,2,3,4,5-pentamethylbenzene.

## Example

The present application is further described in detail below by examples, but the present application is not limited thereto.

## Test Method

### Evaluation of Adsorbent Performance

The adsorption capacity of adsorbent is determined by a toluene gas phase adsorption experiment, wherein the specific operation method is: at 35 °C, nitrogen carrying toluene (toluene partial pressure is 0.05 MPa) is brought into contact with a certain mass of adsorbent until toluene reaches adsorption equilibrium. The adsorption capacity of the adsorbent under test is calculated by the following formula based on the mass difference of the adsorbent before and after toluene adsorption:

$$C = \frac{m_2 - m_1}{m_1} \times 1000$$

wherein, C is the adsorption capacity in mg/g; $m_1$ is the mass of the adsorbent before adsorbing toluene in g; $m_2$ is the mass of the adsorbent after adsorbing toluene in g.

The calcined-base bulk density of the adsorbent is determined by the following method: adding 50 mL of adsorbent into a 100 mL measuring cylinder, vibrating it on a tap density meter (produced by Liaoning Instrument Research Institute Co., Ltd.) for 5 minutes, then adding 50 mL of adsorbent hereinto and vibrating for 5 minutes. The ratio of the mass to the volume of the adsorbent in the measuring cylinder is the adsorbent bulk density; taking a certain mass of adsorbent and calcining it at 600 °C for 2 hours, and placing it in a desiccator to cool to room temperature; the ratio of the mass of the adsorbent after calcination to the mass of the adsorbent before calcination is the calcined-base, and the product of the calcined-base and the adsorbent bulk density is the calcined-base bulk density.

The compressive strength of the adsorbent is represented by the crushing rate of the adsorbent beads under a certain pressure. The lower the crushing rate is, the higher the compressive strength is. The method for measuring the compressive strength of the adsorbent is: a DL-II model particle strength tester (produced by Dalian Chemical Research and Design Institute) is used to conduct the measurement; after passing the adsorbent beads through a 300 microns sieve, about 1.5 mL of adsorbent is loaded into a stainless steel cylinder; when conducting the measurement, a pin with an interference fit with the stainless steel cylinder is installed; after pressing once under a pre-set pressure, the adsorbent is poured out and then passed through a 300-micron sieve and weighed. The mass reduction before and after the pressure testing of adsorbent is the crushing rate of the adsorbent under the set pressure.

The metal ion exchange degree of the adsorbent is determined by the following method: after the ion exchange test, the mass fraction of the metal oxide in the adsorbent is determined by an X-ray fluorescence spectrometer, and the molar fraction of the metal ion is calculated based on this, and the metal ion exchange degree of the adsorbent is further calculated by the following formula:

$$\eta = \frac{\frac{m_2}{M_2}}{\frac{m_1}{M_1} + \frac{m_2}{M_2}} \times 100\%$$

wherein, $\eta$ is the ion exchange degree, $m_1$ is the mass fraction of $Na_2O$ in the adsorbent after ion exchange, $m_2$ is the mass fraction of the target metal (exchanged metal) oxide in the adsorbent after ion exchange, $M_1$ is the molar mass of $Na_2O$, and $M_2$ is the molar mass of the target metal oxide.

Performance Evaluation of Adsorption-desorption System

In the following examples and comparative examples, a dynamic pulse experimental device was used to evaluate the performance of the adsorption-desorption system used, including the adsorption selectivity of the adsorbent and the adsorption and desorption rates of the target product. The device consists of a feeding system, an adsorption column, a heating furnace, a pressure control valve, etc. The adsorption column is a stainless steel tube of $\Phi 6 \times 940$ mm. The lower inlet of the adsorption column is connected to the feeding and nitrogen system, and the upper outlet is connected to the pressure control valve, which is then connected to the effluent collector.

For specific adsorbent and adsorbate, pulse tests were conducted under the same process conditions, different separation effects may be produced by changing the type or composition of the desorbent, because the desorbent, adsorbate, and target component will interact synergistically, promote each other, and restrict each other. The change of the desorbent will change the selectivity difference between the target component and the impurities in the adsorbate, and will also change the selective adsorption of the adsorbent to the target component. Therefore, when using different desorbents to conduct the pulse tests, the peak times, peak shape, intervals, and extreme value positions of the target component and impurities may be different, and the degree of separation may also be different. The specific parameters are manifested as separation coefficients and separation degrees, which will be significantly different.

The measurement method of the adsorption selectivity of the adsorption-desorption system is: loading the adsorbent particles with a particle size of 500-1000 μm into the adsorption column and shake-compacting them, introducing nitrogen at room temperature to exhaust the remaining air in the system, and then introducing the desorbent to remove the gas in the system. Raising the system pressure and temperature to the set values, stopping the introduction of the desorbent, introducing 5-10 mL of pulse feedstock liquid at a volume space velocity of 1.0 $h^{-1}$, wherein the feedstock liquid contains a tracer not to be adsorbed. Then introducing the desorbent at the same volume space velocity, taking 3

drops of desorbed liquid sample every 2 mL, and analyzing it by gas chromatography. With the volume of desorbent used for the desorption as the horizontal axis and the concentration of each component of the pulse feedstock liquid as the vertical axis, drawing the desorption curve of each component of the pulse feedstock liquid. Wherein the tracer not to be adsorbed can be used to obtain the dead volume of the adsorption system. The midpoint of the half-peak width of the tracer is taken as the zero point, and the net retention volume from the midpoint of the half-peak width of each component to the zero point is measured. The net retention volume of any component is proportional to the distribution coefficient at the adsorption equilibrium, reflecting the interaction between each component and the adsorbent material. The ratio of the net retention volumes of the two components is the separation coefficient $\beta$, for example, the ratio of the net retention volume of m-xylene to the net retention volume of ethylbenzene is the ratio of the adsorption performance of the adsorbent in the adsorption-desorption system for the two compounds, which is the separation coefficient of m-xylene relative to ethylbenzene, recorded as $\beta_{MX/EB}$. The larger the β value is, the greater the difference in the adsorption capacity of the adsorbent in the adsorption-desorption system for the two components MX and ethylbenzene (EB) is, that is, MX is more easily to be adsorbed and EB is less easily to be adsorbed, so the two components are easier to be separated.

The half-width of the pulse peak envelope provides information about the mass transfer rate of the fluid between the adsorbent particles, between the molecular sieve grains, and within the molecular sieve crystals; the narrower the half-width of a component is, the smaller the half-width value is, which means the faster the adsorption and desorption rate of the component by the adsorbent in the adsorption-desorption system is, and the faster the mass transfer of the component in the adsorbent is. The increase in the adsorption and desorption rate means the improvement of the adsorbent efficiency, which is conducive to reducing the loading amount of the adsorbent and reducing investment; the increase in the adsorption and desorption rate also means the reduction of the amount of desorbent used for desorption, which is conducive to reducing energy consumption.

Separation degree is usually one of the indicators of separation efficiency of adsorption-desorption system, especially for evaluating the effect of different adsorbents or desorbents on adsorption-separation under the same operating parameters; separation degree is equal to the ratio of the difference between the net retention volumes of two pulse peaks to the average half-peak width of the two pulse peaks, such as the ratio of the difference between the net retention volume of m-xylene and the net retention volume of ethylbenzene to the average half-peak width of the pulse peaks of the two components, that is the separation degree between the two components, which is recorded as $R_{MX/EB}$. According to the definition and calculation method of separation degree, the separation degree of a strongly adsorbed component against a weakly adsorbed component is proportional to the difference in net retention volume between the two components, and inversely proportional to the average half-peak width. Therefore, the separation degree R comprehensively take into accounts of the difference in adsorption strength and the effect of mass transfer rate on the adsorption-separation. The larger the $R_{MX/EB}$ value is, the greater the difference in net retention volume between the MX and EB components is or the smaller the average half-peak width is, the greater the difference in adsorption selectivity is or the faster the adsorption and desorption rate is. Compared with the EB component, MX is easier to be adsorbed on the adsorbent in the adsorption-desorption system or its adsorption and desorption rate in the adsorption-desorption system is faster, and the overall separation effect is better.

In the following examples and comparative examples, unless otherwise specified, all reagents and raw materials used are commercially available products and are chemically pure.

Adsorbent Preparation Example 1

NaY-type molecular sieve with a silicon oxide/aluminum oxide molar ratio of 5.0 and a crystal particle size of 0.8 μm was mixed homogeneously with kaolin mineral and sesbania powder at a mass ratio of 95:4: 1, rolled into beads for molding, and dried, and then calcined at 540 °C for 8 hours. The calcined beads were washed with deionized water, with a liquid-to-solid ratio of 10, and dried at 100 °C for 4 hours to obtain adsorbent A.

The mass ratio of molecular sieve to kaolin mineral in the adsorbent beads was 96: 4; and its X-ray diffraction (XRD) spectrum is shown in Figure 1. Through analysis, the toluene adsorption capacity of adsorbent A was 213 mg/g, the calcined-base bulk density was 0.651 g/mL, the crushing rate at 130N was 0.9 wt%, and the mass fraction of charge-balancing cations calculated as metal oxide was 12.8 %.

Adsorbent Preparation Example 2

NaY-type molecular sieve with a silicon oxide/aluminum oxide molar ratio of 4.3 and a crystal particle size of 0.9 μm was mixed homogeneously with kaolin mineral and sesbania powder at a mass ratio of 95:4: 1, rolled into beads for molding, and dried, and then calcined at 540 °C for 8 hours. The calcined beads were washed with deionized water, with a liquid-to-solid ratio of 10, and dried at 100 °C for 4 hours to obtain adsorbent B.

The mass ratio of molecular sieve to kaolin mineral in the adsorbent beads was 96: 4. Through analysis, the toluene adsorption capacity of adsorbent B was 212 mg/g, the calcined-base bulk density was 0.652 g/mL, the crushing

rate at 130N was 1.0 wt%, and the mass fraction of charge-balancing cations calculated as metal oxide was 13.6 %.

Adsorbent Preparation Example 3

NaY-type molecular sieve with a silicon oxide/aluminum oxide molar ratio of 5.0 and a crystal particle size of 1.1 μm was mixed homogeneously with kaolin mineral and sesbania powder at a mass ratio of 92:7:1, rolled into beads for molding, and dried, and then calcined at 540 °C for 6 hours. The calcined beads were subjected to column ion exchange with 0.25 mol/L silver nitrate solution for 8 hours at a temperature of 85 °C, wherein the liquid-to-solid ratio of the solution to the adsorbent was 45, and the space velocity was 5 $h^{-1}$. The ion exchange degree calculated by the formula was 89.5%. After the exchange, the beads were dried at 100 °C for 3 hours to obtain adsorbent C.

The mass ratio of molecular sieve to kaolin mineral in the adsorbent beads was 93: 7. Through analysis, the toluene adsorption capacity of adsorbent C was 183 mg/g, the calcined-base bulk density was 0.823 g/mL, the crushing rate at 130N was 2.5 wt%, and the mass fraction of charge-balancing cations calculated as metal oxide was 37.6%.

Adsorbent Preparation Example 4

NaY-type molecular sieve with a silicon oxide/aluminum oxide molar ratio of 5.0 and a crystal particle size of 1.0 μm was mixed homogeneously with kaolin mineral and sesbania powder at a mass ratio of 94:5.5:0.5, rolled into beads for molding, and dried, and then calcined at 550 °C for 9 hours. The calcined beads were subjected to column ion exchange with 0.40 mol/L strontium chloride hexahydrate solution for 8 hours at a temperature of 90 °C, wherein the liquid-to-solid ratio of the solution to the adsorbent was 40, and the space velocity was 5$h^{-1}$. The ion exchange degree calculated by the formula was 99.5%, and after exchange, the beads were dried at 100 °C for 4 hours to obtain adsorbent D.

The mass ratio of molecular sieve to kaolin mineral in the adsorbent beads is 94.5:5.5. Through analysis, the toluene adsorption capacity of adsorbent D was 185 mg/g, the calcined-base bulk density was 0.785 g/mL, the crushing rate at 130N was 3.0 wt%, and the mass fraction of the charge-balancing cations calculated as metal oxide was 36.9%.

Adsorbent Preparation Example 5

NaY-type molecular sieve with a silicon oxide/aluminum oxide molar ratio of 5.0 and a crystal particle size of 0.8 μm was mixed homogeneously with kaolin mineral and sesbania powder at a mass ratio of 95:4: 1, rolled into beads for molding, and dried, and then calcined at 550 °C for 9 hours. The calcined beads were subjected to column ion exchange with 0.20 mol/L barium chloride solution for 8 hours at a temperature of 90 °C, wherein the liquid-to-solid ratio of the solution to the adsorbent was 40, and the space velocity was 5$h^{-1}$. The ion exchange degree calculated by the formula was 99.8%, and after exchange, the beads were dried at 100 °C for 4 hours to obtain adsorbent E.

The mass ratio of molecular sieve to kaolin mineral in the adsorbent beads was 95.5:4.5. The toluene adsorption capacity of adsorbent E was analyzed to be 179 mg/g, the calcined-base bulk density was 0.845 g/mL, the crushing rate at 130N was 3.0 wt%, and the mass fraction of the charge-balancing cations calculated as metal oxide was 39.8 %.

Example 1

An appropriate amount of adsorbent A was taken and programmed to be heated to 180 °C in a muffle furnace, then was subjected to fluidized dehydration for 2 hours; the mass fraction of adsorbed water thereof was determined as 1.84%. 26 ml of the adsorbent was taken to conduct a liquid pulse experiment to determine its adsorption selectivity, separation degree, and the adsorption and desorption rates of m-xylene. The pressure of the liquid pulse experiment was 0.8 MPa, the temperature was 145 °C, and the desorbent used in the experiment was 30 wt% of 1,2,3-trimethylbenzene and 70 wt% of n-heptane. The composition of the pulse feedstock liquid was 5 wt% of p-xylene, m-xylene, o-xylene, ethylbenzene, n-nonane and 75 wt% of desorbent, wherein n-nonane was a tracer.

The separation coefficient and separation degree between m-xylene and ethylbenzene, p-xylene and o-xylene ($\beta_{MX/EB}$ and $R_{MX/EB}$, $\beta_{MX/PX}$ and $R_{MX/PX}$, $\beta_{MX/OX}$ and $R_{MX/OX}$) are shown in Table 1, and the pulse spectrum is shown in Figure 2.

Example 2

M-xylene in the mixed C8 aromatic hydrocarbons was separated according to the method of Example 1, except that the adsorbent A was programmed to be heated to 220 °C in a muffle furnace and then subjected to fluidized dehydration for 2 hours; the mass fraction of adsorbed water thereof was measured to be 1.03%.

The results of the separation coefficient and separation degree between m-xylene and ethylbenzene, p-xylene and o-xylene are shown in Table 1.

Example 3

M-xylene in the mixed C8 aromatic hydrocarbons was separated according to the method of Example 1, except that the desorbent used in the experiment was 50 wt% of 1,2,3-trimethylbenzene and 50 wt% of n-heptane.
The results of the separation coefficient and separation degree between m-xylene and ethylbenzene, p-xylene and o-xylene are shown in Table 1.

Example 4

M-xylene in the mixed C8 aromatic hydrocarbons was separated according to the method of Example 1, except that the desorbent used in the experiment was 80 wt% of 1,2,3-trimethylbenzene and 20 wt% of n-heptane.
The results of the separation coefficient and separation degree between m-xylene and ethylbenzene, p-xylene and o-xylene are shown in Table 1.

Example 5

M-xylene in the mixed C8 aromatic hydrocarbons was separated according to the method of Example 2, except that the desorbent was 1,2,3-trimethylbenzene.
The results of the separation coefficient and separation degree between m-xylene and ethylbenzene, p-xylene and o-xylene are shown in Table 1.

Example 6

M-xylene in the mixed C8 aromatic hydrocarbons was separated according to the method of Example 1, except that the adsorbent A was programmed to be heated to 230 °C in a muffle furnace and then subjected to fluidized dehydration for 2 hours; the mass fraction of adsorbed water thereof was determined to be 0.82%.
The results of the separation coefficient and separation degree between m-xylene and ethylbenzene, p-xylene and o-xylene are shown in Table 1, and the pulse spectrum is shown in Figure 3.

Example 7

M-xylene in the mixed C8 aromatic hydrocarbons was separated according to the method of Example 1, except that the adsorbent A was programmed to be heated to 250 °C in a muffle furnace and then subjected to fluidized dehydration for 2 hours; the mass fraction of adsorbed water thereof was measured to be 0.55%.
The results of the separation coefficient and separation degree between m-xylene and ethylbenzene, p-xylene and o-xylene are shown in Table 1.

Example 8

M-xylene in the mixed C8 aromatic hydrocarbons was separated according to the method of Example 1, except that the adsorbent A was programmed to be heated to 280 °C in a muffle furnace and then subjected to fluidized dehydration for 2 hours, and the mass fraction of adsorbed water thereof was determined to be 0.10 %.
The results of the separation coefficient and separation degree between m-xylene and ethylbenzene, p-xylene and o-xylene are shown in Table 1.

Example 9

M-xylene in the mixed C8 aromatic hydrocarbons was separated according to the method of Example 1, except that the adsorbent A was programmed to be heated to 260 °C in a muffle furnace and then subjected to fluidized dehydration for 2 hours; the mass fraction of adsorbed water thereof was determined to be 0.26%.
The results of the separation coefficient and separation degree between m-xylene and ethylbenzene, p-xylene and o-xylene are shown in Table 1, and the pulse spectrum is shown in Figure 4.

Example 10

M-xylene in the mixed C8 aromatic hydrocarbons was separated according to the method of Example 9, except that the temperature of the liquid phase pulse experiment was 130 °C.
The results of the separation coefficient and separation degree between m-xylene and ethylbenzene, p-xylene

and o-xylene are shown in Table 1, and the pulse spectrum is shown in Figure 5.

Example 11

M-xylene in the mixed C8 aromatic hydrocarbons was separated according to the method of Example 1, except that the adsorbent was adsorbent B.

The results of the separation coefficient and separation degree between m-xylene and ethylbenzene, p-xylene and o-xylene are shown in Table 1.

Example 12

M-xylene in the mixed C8 aromatic hydrocarbons was separated according to the method of Example 1, except that the adsorbent was adsorbent C.

The results of the separation coefficient and separation degree between m-xylene and ethylbenzene, p-xylene and o-xylene are shown in Table 1.

Example 13

M-xylene in the mixed C8 aromatic hydrocarbons was separated according to the method of Example 1, except that the adsorbent was adsorbent D.

The results of the separation coefficient and separation degree between m-xylene and ethylbenzene, p-xylene and o-xylene are shown in Table 1.

Example 14

M-xylene in the mixed C8 aromatic hydrocarbons was separated according to the method of Example 9, except that the desorbent was 20% of tetratoluene and 80 % of n-octane.

The results of the separation coefficient and separation degree between m-xylene and ethylbenzene, p-xylene and o-xylene are shown in Table 1.

Embodiment 15

M-xylene in the mixed C8 aromatic hydrocarbons was separated according to the method of Example 9, except that the desorbent was 50% of pentatoluene and 50% of n-decane.

The results of the separation coefficient and separation degree between m-xylene and ethylbenzene, p-xylene and o-xylene are shown in Table 1.

Example 16

M-xylene in the mixed C8 aromatic hydrocarbons was separated by the method of Example 9 except that the desorbent was 50% of 3-ethyl-o-xylene and 50% of n-undecane were present.

The results of the separation coefficient and separation degree between m-xylene and ethylbenzene, p-xylene and o-xylene are shown in Table 1.

Example 17

Meta-dimethylnaphthalene in the mixed C12 aromatic hydrocarbons was separated according to the method of Example 1, except that the adsorbent was E, and the composition of the pulse feedstock liquid was 5 wt% of 2,7-dimethylnaphthalene, 5 wt% of 1,6-dimethylnaphthalene, 5 wt% of 2,6-dimethylnaphthalene, 5 wt% of 1,8-dimethylnaphthalene, 5 wt% of n-nonane and 75 wt% of desorbent, wherein n-nonane was a tracer.

The results of the separation coefficient and separation degree between 2,7-dimethylnaphthalene and 1,6-dimethylnaphthalene, 2,6-dimethylnaphthalene and 1,8-dimethylnaphthalene are shown in Table 1.

Example 18

Meta-dimethylnaphthalene in the mixed C12 aromatic hydrocarbons was separated according to the method of Example 17, except that when the adsorbent was subjected to ion exchange experiment, the concentration of the exchange liquid was 0.15 mol/L, and the exchange degree of the obtained adsorbent E was 78.5%.

The results of the separation coefficient and separation degree between 2,7-dimethylnaphthalene and 1,6-dimethylnaphthalene, 2,6-dimethylnaphthalene and 1,8-dimethylnaphthalene are shown in Table 1.

Example 19

M-xylene in mixed C8 aromatic hydrocarbons was separated according to the method of Example 9, except that the composition of the pulse feedstock liquid was 10 wt% of m-xylene, 10 wt% of ethylbenzene, 10 wt% of n-nonane and 70 wt% of a desorbent, wherein n-nonane was a tracer.
The results of the separation coefficient and separation degree between m-xylene and ethylbenzene are shown in Table 1.

Comparative Example 1

M-xylene in the mixed C8 aromatic hydrocarbons was separated according to the method of Example 9, except that the desorbent was 30 wt% of toluene and 70 wt% of n-heptane.
The results of the separation coefficient and separation degree between m-xylene and ethylbenzene, p-xylene and o-xylene are shown in Table 1, and the pulse spectrum is shown in Figure 6.

Comparative Example 2

M-xylene in the mixed C8 aromatic hydrocarbons was separated according to the method of Example 9, except that the desorbent was 30 wt% of tetralin and 70 wt% of n-heptane.
The results of the separation coefficient and separation degree between m-xylene and ethylbenzene, p-xylene and o-xylene are shown in Table 1, and the pulse spectrum is shown in Figure 7.

Comparative Example 3

M-xylene in the mixed C8 aromatic hydrocarbons was separated according to the method of Example 9, except that the desorbent was 30 wt% of 1,2,4-trimethylbenzene and 70 wt% of n-heptane.
The results of the separation coefficient and separation degree between m-xylene and ethylbenzene, p-xylene and o-xylene are shown in Table 1, and the pulse spectrum is shown in Figure 8.

Comparative Example 4

M-xylene in the mixed C8 aromatic hydrocarbons was separated according to the method of Example 1, except that the adsorbent A was programmed to be heated to 600 °C in a muffle furnace and dehydrated for 24 hours; the mass fraction of adsorbed water thereof was determined to be 0.005%.
The results of the separation coefficient and separation degree between m-xylene and ethylbenzene, p-xylene and o-xylene are shown in Table 1.

Comparative Example 5

M-xylene in the mixed C8 aromatic hydrocarbons was separated according to the method of Example 9, except that the adsorption temperature was 95 °C.
The results of the separation coefficient and separation degree between m-xylene and ethylbenzene, p-xylene and o-xylene are shown in Table 1.

Comparative Example 6

M-xylene in the mixed C8 aromatic hydrocarbons was separated according to the method of Example 9, except that the desorbent was 10 wt% of 1,2,3-trimethylbenzene and 90 wt% of n-heptane.
The results of the separation coefficient and separation degree between m-xylene and ethylbenzene, p-xylene and o-xylene are shown in Table 1.

Table 1. Test results of Examples 1-18 and Comparative Examples 1-6

| No. | Adsorbent | NaY-type molecular sieve ion exchange degree, mol% | Water content of adsorbent, wt% | Desorbent alkane content, wt% | Separation coefficient | | | Separation degree | | |
|---|---|---|---|---|---|---|---|---|---|---|
| | | | | | $\beta MX/_{EB}$ $\beta_{2,7/1,6}$ | $\beta MX/_{PX}$ $\beta_{2,7/2,6}$ | $\beta MX/_{O-X}$ $\beta_{2,7/1,8}$ | RMX/EB $R_{/2,7/1,6}$ | $R_{MX/PX}$ $R_{2,7/2,6}$ | $R_{MX/OX}$ $R_{2,7/1,8}$ |
| Example 1 | A | 0 | 1.84 | 70 | 2.24 | 2.46 | 2.26 | 0.74 | 0.79 | 0.71 |
| Example 2 | A | 0 | 1.03 | 70 | 2.42 | 2.65 | 2.34 | 0.76 | 0.81 | 0.72 |
| Example 3 | A | 0 | 1.03 | 50 | 2.41 | 2.63 | 2.32 | 0.77 | 0.82 | 0.73 |
| Example 4 | A | 0 | 1.03 | 20 | 2.39 | 2.58 | 2.31 | 0.75 | 0.81 | 0.72 |
| Example 5 | A | 0 | 1.03 | 0 | 2.23 | 2.44 | 2.20 | 0.74 | 0.80 | 0.70 |
| Example 6 | A | 0 | 0.82 | 70 | 2.45 | 2.78 | 2.54 | 0.85 | 0.94 | 0.85 |
| Example 7 | A | 0 | 0.55 | 70 | 2.64 | 3.15 | 2.58 | 0.94 | 1.05 | 0.87 |
| Example 8 | A | 0 | 0.26 | 70 | 2.71 | 3.32 | 2.78 | 0.96 | 1.09 | 0.94 |
| Example 9 | A | 0 | 0.26 | 70 | 2.87 | 3.39 | 2.80 | 0.93 | 1.00 | 0.86 |
| Example 10 | A | 0 | 0.10 | 70 | 2.67 | 3.19 | 2.60 | 0.94 | 1.06 | 0.88 |
| Example 11 | B | 0 | 1.82 | 70 | 2.30 | 2.42 | 2.15 | 0.79 | 0.75 | 0.68 |
| Example 12 | C | 89.5 | 1.80 | 70 | 2.20 | 2.35 | 2.28 | 0.70 | 0.74 | 0.77 |
| Example 13 | D | 99.5 | 1.84 | 70 | 2.26 | 2.41 | 2.19 | 0.75 | 0.76 | 0.69 |
| Example 14 | A | 0 | 0.26 | 80 | 2.66 | 3.31 | 2.70 | 0.95 | 1.08 | 0.91 |
| Example 15 | A | 0 | 0.26 | 50 | 2.40 | 2.78 | 2.46 | 0.80 | 0.85 | 0.73 |
| Example 16 | A | 0 | 0.26 | 50 | 2.26 | 2.64 | 2.30 | 0.75 | 0.78 | 0.72 |
| Example 17 | E | 99.8 | 0.56 | 70 | 2.94 | 7.54 | 2.50 | 1.36 | 2.40 | 1.23 |
| Example 18 | E | 78.5 | 0.56 | 70 | 2.32 | 5.95 | 1.97 | 1.06 | 1.85 | 0.96 |
| Example 19 | A | 0 | 0.26 | 70 | 2.53 | / | / | 0.96 | / | / |
| Comparative Example 1 | A | 0 | 0.26 | 70 | 4.50 | 2.00 | 2.05 | 0.97 | 0.67 | 0.68 |
| Comparative Example 2 | A | 0 | 0.26 | 70 | 2.41 | 1.52 | 1.61 | 0.69 | 0.39 | 0.42 |
| Comparative Example 3 | A | 0 | 0.26 | 70 | 2.00 | 1.76 | 1.60 | 0.68 | 0.53 | 0.46 |
| Comparative Example 4 | A | 0 | 0.005 | 70 | 2.20 | 2.44 | 2.11 | 0.54 | 0.55 | 0.50 |
| Comparative Example 5 | A | 0 | 0.26 | 70 | 2.10 | 2.40 | 2.13 | 0.69 | 0.70 | 0.68 |

(continued)

| No. | Adsorbent | NaY-type molecular sieve ion exchange degree, mol% | Water content of adsorbent, wt% | Desorbent alkane content, wt% | Separation coefficient | | | Separation degree | | |
|---|---|---|---|---|---|---|---|---|---|---|
| | | | | | $\beta MX/_{EB}$ $\beta_{2,7/1,6}$ | $\beta MX/_{PX}$ $\beta_{2,7/2,6}$ | $\beta MX/_{O-X}$ $\beta_{2,7/1,8}$ | RMX/EB $R_{/2,7/1,6}$ | $R_{MX/PX}$ $R_{2,7/2,6}$ | $R_{MX/OX}$ $R_{2,7/1,8}$ |
| Comparative Example 6 | A | 0 | 0.26 | 90 | 1.55 | 1.80 | 1.57 | 0.55 | 0.65 | 0.54 |

It can be seen from the test results in Table 1 that in Examples 1-13 of the present application, when 1,2,3-trimethylbenzene or its mixture with alkanes is used as a desorbent for adsorption-separation of MX, the separation effect is improved compared with toluene as a desorbent (Example 1 vs. Comparative Example 1). Ethylbenzene (EB), p-xylene (PX), and o-xylene (OX) components other than MX can be separated from MX at the same time; the net retention volumes corresponding to the pulse peaks of the three components are relatively similar, and the separation coefficients and separation degrees of the three components with MX are slightly different, indicating that the interaction forces between the three impurities and the adsorbent are similar, which is significantly different from the interaction force between the target component and the adsorbent. Therefore, the three impurities can be removed in the same time period and the same process section by process parameter design, while when toluene is used as a desorbent, the three impurities cannot be removed in the same time period and the same process section. If they are forcibly removed at the same time, the process parameter control range is very small, and the precision requirements for the process device are high. Therefore, when toluene is used as a desorbent, the efficiency of the entire separation process is lower, the material consumption is larger, and the energy consumption for recycling is also higher. In addition, in this adsorption-separation system, the components that are difficult to be separated from MX are PX and OX, and the component that is easier to be separated is EB. Therefore, whether a highly-pure MX product can be obtained depends on the complete removal of the impurities PX and OX, which are the most difficult to be separated, that is, it is necessary to compare the separation coefficient and separation degree between these two components and MX, rather than focusing on the removal of easily separated impurities. Compared with the case that toluene is used as a desorbent, the net retention volume corresponding to the pulse peaks of PX and OX is significantly reduced, indicating that in the presence of 1,2,3-trimethylbenzene desorbent, the adsorbent's adsorption capacity for PX and OX is significantly weakened, and the separation of PX and OX from MX is easier than when toluene is used as a desorbent; the separation coefficient and separation degree thereof are larger, the separation effect thereof is better, the speed of the entire separation process is faster, the usage amount of materials is smaller, and the energy consumption for recycling is lower.

The test results of Examples 14-18 show that the present application can also be applied for the separation of other meta-aromatic hydrocarbon(s), such as meta-dimethylnaphthalene, and other alkylbenzene compounds conforming to the general formula (I), such as tetratoluene, pentatoluene and 3-ethyl-o-xylene, can also be used in the present application.

Example 20

Adsorbent A was used, with an adsorbent loading of 2116 g and a water content of 0.26%. A simulated moving bed adsorption-separation device was used to separate m-xylene in the mixed C8 aromatic hydrocarbons. Wherein the mixed C8 aromatic hydrocarbons feedstock contained 1.5 wt% of non-aromatics, 0.5 wt% of benzene, 0.9 wt% of toluene, 48.1 wt% of m-xylene, 20.2 wt% of p-xylene, 18.9 wt% of o-ylene, 9.8 wt% of ethylbenzene, and 0.1 wt% of C9+ aromatics.

The simulated moving bed device was composed of 24 columns connected in series. The cavity inside the column for accommodating the adsorbent was 200 mm high and 40 mm in diameter. The 24th column was connected to the first column through a pump to circulate the fluid in the column. The materials could be introduced or discharged at connection position of each column. There were 7 columns between the raffinate outlet and the feedstock inlet, which was the adsorption zone; there were 9 columns between the feedstock inlet and the extract liquid outlet, which was the purification zone; there were 5 columns between the extract liquid outlet and the desorbent inlet, which was the desorption zone; there were 3 columns between the desorbent inlet and the raffinate outlet, which was the isolation zone. The positions of the inlet and outlet of each material stream are shown in Figure 9. The inlet and outlet positions of the material changed according to the step time. The inlet and outlet were pushed forward by one column every step time. The inlet and outlet were moved from the solid arrow position in the figure to the dotted arrow position; the next step time moved forward in the established direction; the positions of the inlet and outlet were changed in sequence by similar mode until the inlet and outlet returned to the starting position, which is a cycle. One step time is 80 seconds and one cycle is 32 minutes. After the simulated moving bed ran stably, mixed sample of the extract and mixed sample of the raffinate were taken for one cycle and their compositions were analyzed. According to the analysis results, the calculation method of the purity and yield of m-xylene is:

$$\text{Purity of m-xylene} = \frac{X_{\text{m-xylene}}}{X_{\text{non aromatic}} + X_{\text{benzene}} + X_{\text{toluene}} + X_{\text{m-xylene}} + X_{\text{p-xylene}} + X_{\text{o-xylene}} + X_{\text{ethylbenzene}} + X_{\text{C9+aromatics}}} \times 100\%$$

wherein X is the mass fraction of each component in the extract liquid;

$$\text{M-xylene yield} = \frac{X_{\text{m-xylene,extract}} \times Q_{\text{extract}}}{X_{\text{m-xylene,extract}} \times Q_{\text{extract}} + X_{\text{m-xylene,raffinate}} \times Q_{\text{raffinate}}} \times 100\%$$

wherein $x_{m\text{-}xylene,extract}$ is the mass fraction of m-xylene in the extract liquid, $Q_{extract}$ is the mass flow rate of the extract liquid, $X_{m\text{-}xylene,raffinate}$ is the mass fraction of m-xylene in the raffinate, and $Q_{raffinate}$ is the mass flow rate of the raffinate.

The temperature of adsorption bed layer was controlled at 145 °C and the operating pressure was 0.90 MPa. The desorbent was 93 wt% 1,2,3-trimethylbenzene (purchased from Xilong Chemical Reagent Company, CAS: 526-73-8), the feedstock feed rate was 0.36 kg/h, the desorbent injection rate was 1.79 kg/h, the extract liquid rate was 0.84 kg/h, the raffinate liquid rate was 1.31 kg/h, and the regional flow rate of the purification zone was 2.85 kg/h. The mass flow rate ratio of the desorbent to the C8 aromatic hydrocarbons feedstock entering the simulated moving bed was 4.97, and the C8 aromatic hydrocarbons feedstock rate per unit mass of adsorbent was 0.17 kg/(h·kg adsorbent). The number of adsorption bed layers in the simulated moving bed adsorption-separation device was 24, and the number of bed layers in the adsorption zone, purification zone, desorption zone and isolation zone were 7, 9, 5, and 3, respectively. The step time was 80 seconds, and one cycle period was 32 minutes. After adsorption-separation, the yield of m-xylene was 96.25 wt% and the purity was 99.65 wt%. The desorbent accounted for 81.11 wt% in the extract and the desorbent accounted for 84.23 wt% in the raffinate. According to the compositions of the extract and of raffinate, the number of theoretical plates, the top condensation load and the bottom heating load of the distillation tower were calculated by using the RadFrac module of Aspen Plus software. According to an industrial adsorption-separation device with an annual production capacity of 200,000 tons of m-xylene, the desorbent 1,2,3-trimethylbenzene was recovered at the top of the extract tower in the extract and at raffinate tower kettle respectively. The simulation calculation results showed that the extract flow rate was 124 t/h and the raffinate flow rate was 202 t/h, the mass fraction of the recovered desorbent after separation can reach 99.995%, the mass fraction of the desorbent in the M-xylene product at the top of the tower or other C8 aromatic hydrocarbons was 0.01%, and the heat loads of the extract distillation tower and of the raffinate distillation tower kettle were 11.8 Gcal/h and 18.2 Gcal/h, respectively.

Comparative Example 7

M-xylene in mixed C8 aromatic hydrocarbons was separated according to the method of Example 20, except that the desorbent was 99 wt% toluene (purchased from Inokai Technology Co., Ltd., CAS: 108-88-3). Under the same operating conditions, the regional flow rate of the purification zone was 2.90 kg/h, the yield of m-xylene was 95.55 wt%, and the purity was 99.50 wt%. The desorbent toluene was recovered at the top of the extract distillation tower and the raffinate distillation tower, respectively. The simulation calculation results showed that the heat loads of the extract distillation tower and the raffinate distillation tower were 27.6 Gcal/h and 35.8 Gcal/h, respectively.

By comparing the separation purity and yield results of Example 20 and Comparative Example 7, it can be seen that when 1,2,3-trimethylbenzene was used as a desorbent, the separation of the components which are more difficult to be separated (PX and OX) from the target component MX is easier than when toluene was used as a desorbent, which is beneficial to the purification of MX in the purification zone of the simulated moving bed. At the same time, the required regional flow rate of the purification zone is reduced to a certain extent, about 10 %, that is, the reduction in material consumption, which saves the cost of purchasing materials and recycling costs. The purity and yield of the final target component MX are correspondingly improved, the purity is increased by 0.1 percentage points, and the yield is increased by 0.75 percentage points, which will bring about the improvement of MX separation efficiency in industrial devices, the increase in the amount of processed feedstock, and the reduction of operating costs. The boiling point of 1,2,3-trimethylbenzene is higher than that of the target component MX; when it is used as a desorbent, it is recovered in the kettle of the extract and raffinate distillation towers for continuous recycling. Therefore, compared with when toluene was used as a desorbent and recovered at the top of the tower, the required tower kettle heat load is significantly reduced, the heat load of the extract distillation tower is reduced by 57 %, the heat load of the raffinate distillation tower is reduced by 49 %, and the energy consumption of the device is significantly reduced.

The preferred embodiments of the present application are described in detail above; however, the present application is not limited to the specific details in the above embodiments. Within the technical concept of the present application, a variety of simple modifications can be made to the technical solution of the present application, and these simple modifications all fall within the protection scope of the present application.

It should also be noted that the various specific technical features described in the above specific embodiments

can be combined in any suitable manner without contradiction. In order to avoid unnecessary repetition, this application will not further describe various possible combinations.

In addition, the various embodiments of the present application may be arbitrarily combined, and as long as they do not violate the concept of the present application, they should also be regarded as the content invented by the present application.

## Claims

1. Use of a liquid material comprising or consisting of 20-100 wt% of an alkylbenzene compound of the following general formula (I) and 0-80 wt% of a C5-C14 saturated aliphatic hydrocarbons as a desorbent for the adsorption-separation of meta-aromatic hydrocarbon(s),

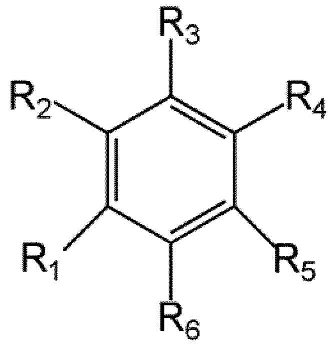

(I),

wherein:

$R_1$, $R_2$ and $R_3$, which may be the same or different, are independently selected from $C_{1-4}$ chain alkyl groups, preferably, at least one of $R_1$, $R_2$ and $R_3$ is methyl, more preferably at least two of them are methyl, and further preferably all of them are methyl;

$R_4$, $R_5$ and $R_6$, which may be the same or different, are independently selected from hydrogen, $C_{1-4}$ saturated hydrocarbon group, $C_{1-4}$ alkoxy group and halogen; preferably, at least one of $R_4$, $R_5$ and $R_6$ is hydrogen or $C_{1-4}$ saturated hydrocarbon group, for example, $R_4$ or $R_6$ is $C_{1-4}$ saturated hydrocarbon group, more preferably, at least two of them are independently hydrogen or $C_{1-4}$ saturated hydrocarbon group, for example, $R_4$ and $R_6$ are both $C_{1-4}$ saturated hydrocarbon group, further preferably, three of them are independently hydrogen or $C_{1-4}$ saturated hydrocarbon group, and particularly preferably, all three are hydrogen, wherein the $C_{1-4}$ saturated hydrocarbon group is preferably methyl group.

2. The use according to claim 1, wherein the adsorption-separation comprises separating the meta-aromatic hydrocarbon(s) from a mixed aromatic hydrocarbon feedstock comprising the meta-aromatic hydrocarbon(s) and at least one isomer thereof by adsorption and desorption, wherein the meta-aromatic hydrocarbon(s) are preferably C8-C12 meta-aromatic hydrocarbon(s), more preferably C8-C12 meta-alkyl aromatic hydrocarbons, such as m-xylene or 2,7-dimethylnaphthalene,

further preferably, the mixed aromatic hydrocarbon feedstock is a mixed C8 aromatic hydrocarbon feedstock comprising m-xylene and at least one other C8 aromatic hydrocarbon selected from p-xylene, o-xylene and ethylbenzene;

more preferably, the mixed C8 aromatic hydrocarbon feedstock contains 5-95 wt% of m-xylene.

3. The use according to claim 2, wherein the meta-aromatic hydrocarbon is m-xylene, and the mixed aromatic hydrocarbon feedstock comprises 5-94 wt% of m-xylene and 6-95 wt% of p-xylene, preferably comprises 5-90 wt% of m-xylene and 10-95 wt% of p-xylene.

4. The use according to any one of claims 2 to 3, wherein the adsorbent used in the adsorption-separation comprises at least 90 wt% of a Y-type molecular sieve as an active component, the Y-type molecular sieve having a silicon oxide/aluminum oxide molar ratio of 4.0-6.0, preferably 4.3-5.7,

further preferably, the adsorbent comprises one or more, for example one or two, of groups IA, IIA and IB metal ions as charge-balancing cations, wherein the group IA metal ions are preferably selected from $Li^+$ and $Na^+$, the group IIA metal ions are preferably selected from $Mg^{2+}$, $Sr^{2+}$ and $Ba^{2+}$, and the group IB metal ions are preferably $Ag^+$, preferably, the total amount of groups IA, IIA and IB metals in the adsorbent, calculated as metal oxide and based on the total weight of the adsorbent, is 10-45%, preferably 10-40 %;
more preferably, the adsorbent contains 0.05-2 wt%, preferably 0.05-1 wt%, more preferably 0.05-0.8 wt%, and further preferably 0.1-0.5 wt% of adsorbed water;
particularly preferably, the adsorbent contains at least 90 wt% of NaY-type molecular sieve as an active component, and is preferably treated with metal ion exchange to have a metal ion exchange degree of 78.0-99.9%.

**5.** The use according to any one of claims 2 to 4, wherein, based on the total amount of the liquid material, the liquid material comprises or consists of 30-95 wt%, preferably 30-80 wt%, more preferably 30-50 wt% of the alkylbenzene compound, and 5-70 wt%, preferably 20-70 wt%, more preferably 50-70 % of alkanes selected from C5-C14 normal alkanes, preferably selected from C6-C10 normal alkanes.

**6.** The use according to any one of claims 2 to 5, wherein in formula (I), at least two of $R_1$, $R_2$ and $R_3$ are methyl, $R_4$, $R_5$ and $R_6$ are independently hydrogen or $C_{1-4}$ saturated hydrocarbon groups, and at least one of them is hydrogen; preferably, at least two of $R_4$, $R_5$ and $R_6$ are hydrogen, and the $C_{1-4}$ saturated hydrocarbon group is methyl;
more preferably, the alkylbenzene compound is selected from 1,2,3-trimethylbenzene, 1,2,3,4-tetramethylbenzene, 1,2,3,4,5-pentamethylbenzene and 3-ethyl-o-xylene, preferably 1,2,3-trimethylbenzene or 1,2,3,4-tetramethylbenzene.

**7.** The use according to claim 4, wherein the meta-aromatic hydrocarbon is m-xylene, the mixed aromatic hydrocarbon feedstock comprises 20-60 wt% of m-xylene, 10-30 wt% of p-xylene, 10-30 wt% of o-xylene and 5-20 wt% of ethylbenzene, and the alkylbenzene compound is selected from 1,2,3-trimethylbenzene, 1,2,3,4-tetramethylbenzene, 1,2,3,4,5-pentamethylbenzene and 3-ethyl-o-xylene;

based on the total amount of the liquid material, the liquid material comprises 30-70 wt% of the alkylbenzene compound and 30-70 wt% of an alkane selected from C5-C14 normal alkanes; and
the charge-balancing cations of the adsorbent are selected from $Na^+$, $Sr^{2+}$, $Ba^{2+}$ and $Ag^+$, and the adsorbent contains 0.05-0.8 wt%, preferably 0.1-0.5 wt% of adsorbed water.

**8.** A method for separating meta-aromatic hydrocarbon(s) from a mixed aromatic hydrocarbon feedstock comprising the meta-aromatic hydrocarbon(s) and isomers thereof, the method comprising the steps of:

1) the mixed aromatic hydrocarbon feedstock is contacted with an adsorbent to adsorb the meta-aromatic hydrocarbon(s), thereby obtaining an adsorbent adsorbed with the meta-aromatic hydrocarbon(s) and a raffinate containing non-adsorbed components;
2) the adsorbent adsorbed with the meta-aromatic hydrocarbon(s) obtained in step 1) is contacted with a desorbent to desorb the meta-aromatic hydrocarbon(s), to obtain an extract liquid containing the meta-aromatic hydrocarbon(s) and the desorbent; and
3) the extract liquid obtained in step 2) is subjected to a rectification-separation to obtain the meta-aromatic hydrocarbon(s) and the desorbent,

wherein, based on the total amount of the desorbent, the desorbent comprises or consists of 20-100 wt% of an alkylbenzene compound of the following general formula (I) and 0-80 wt% of a C5-C14 saturated aliphatic hydrocarbon(s),

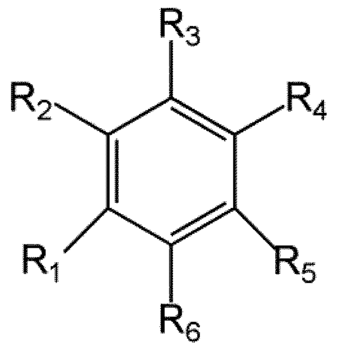

(I),

wherein:

$R_1$, $R_2$ and $R_3$, which may be the same or different, are independently selected from $C_{1-4}$ chain alkyl groups, preferably, at least one of $R_1$, $R_2$ and $R_3$ is methyl, more preferably at least two of them are methyl, and further preferably all of them are methyl;

$R_4$, $R_5$ and $R_6$, which may be the same or different, are independently selected from hydrogen, $C_{1-4}$ saturated hydrocarbon group, $C_{1-4}$ alkoxy group and halogen, preferably, at least one of $R_4$, $R_5$ and $R_6$ is hydrogen or $C_{1-4}$ saturated hydrocarbon group, for example, $R_4$ or $R_6$ is $C_{1-4}$ saturated hydrocarbon group, more preferably, at least two of them are independently hydrogen or $C_{1-4}$ saturated hydrocarbon group, for example, $R_4$ and $R_6$ are both $C_{1-4}$ saturated hydrocarbon group, further preferably, three of them are independently hydrogen or $C_{1-4}$ saturated hydrocarbon group, and particularly preferably, all three are hydrogen, wherein the alkyl group is preferably methyl.

**9.** The method according to claim 8, wherein the meta-aromatic hydrocarbon is a C8-C12 meta-aromatic hydrocarbon, preferably a C8-C12 meta-alkyl aromatic hydrocarbon, such as m-xylene or 2,7-dimethylnaphthalene,
preferably, the mixed aromatic hydrocarbon feedstock is a mixed C8 aromatic hydrocarbon feedstock comprising m-xylene and at least one other C8 aromatic selected from p-xylene, o-xylene and ethylbenzene, and further preferably, the mixed C8 aromatic hydrocarbon feedstock comprises 5-95 wt% of m-xylene.

**10.** The method according to claim 9, wherein the meta-aromatic hydrocarbon is m-xylene, and the mixed aromatic hydrocarbon feedstock comprises 5-94 wt% of m-xylene and 6-95 wt% of p-xylene, preferably 5-90 wt% of m-xylene and 10-95 wt% of p-xylene.

**11.** The method according to any one of claims 8 to 10, wherein, based on the total amount of the desorbent, the desorbent comprises or consists of 30-95 wt%, preferably 30-80 wt%, more preferably 30-50 wt% of the alkylbenzene compound, and 5-70 wt%, preferably 20-70 wt%, more preferably 50-70 wt% of alkanes selected from C5-C14 normal alkanes, preferably selected from C6-C10 normal alkanes.

**12.** The method according to any one of claims 8 to 11, wherein in formula (I), at least two of $R_1$, $R_2$ and $R_3$ are methyl, $R_4$, $R_5$ and $R_6$ are independently hydrogen or a $C_{1-4}$ saturated hydrocarbon group, and at least one of them is hydrogen; preferably, at least two of $R_4$, $R_5$ and $R_6$ are hydrogen, and the $C_{1-4}$ saturated hydrocarbon group is methyl;
more preferably, the alkylbenzene compound is selected from 1,2,3-trimethylbenzene, 1,2,3,4-tetramethylbenzene, 1,2,3,4,5-pentamethylbenzene and 3-ethyl-o-xylene, preferably 1,2,3-trimethylbenzene or 1,2,3,4-tetramethylbenzene.

**13.** The method according to any one of claims 8 to 12, wherein the adsorbent used in step 1) comprises at least 90 wt% of a Y-type molecular sieve as an active component, the Y-type molecular sieve having a silicon oxide/aluminum oxide molar ratio of 4.0-6.0, preferably 4.3-5.7,

preferably, the adsorbent comprises one or more, for example one or two, of groups IA, IIA and IB metal ions as charge-balancing cations, wherein the group IA metal ions are preferably selected from $Li^+$ and $Na^+$, the group IIA metal ions are preferably selected from $Mg^{2+}$, $Sr^{2+}$ and $Ba^{2+}$, and the group IB metal ions are preferably $Ag^+$, preferably, the total amount of groups IA, IIA and IB metals in the adsorbent, calculated as metal oxide and based on the total amount of the adsorbent, is 10-45%,
more preferably, the crystal size of the Y-type molecular sieve is 0.5-2.0 μm, preferably 0.8-1.2 μm,

more preferably, the adsorbent comprises at least 90 wt% of NaY-type molecular sieve as an active component, and is preferably treated with metal ion exchange to obtain a metal ion exchange degree of 78.0-99.9%.

**14.** The method according to claim 13, wherein the adsorbent contains 0.05-2 wt%, preferably 0.05-1 wt%, more preferably 0.05-0.8 wt%, further preferably 0.1-0.5 wt% of adsorbed water.

**15.** The method according to claim 13, wherein the meta-aromatic hydrocarbon is m-xylene, the mixed aromatic hydrocarbon feedstock comprises 20-60 wt% of m-xylene, 10-30 wt% of p-xylene, 10-30 wt% of o-xylene and 5-20 wt% of ethylbenzene, and the alkylbenzene compound is selected from 1,2,3-trimethylbenzene, 1,2,3,4-tetramethylbenzene, 1,2,3,4,5-pentamethylbenzene and 3-ethyl-o-xylene;

based on the total amount of the liquid material, the liquid material comprises 30-70 wt% of the alkylbenzene compound and 30-70 wt% of an alkane selected from C5-C14 normal alkanes; and
the charge-balancing cations of the adsorbent are selected from $Na^+$, $Sr^{2+}$, $Ba^{2+}$ and $Ag^+$, and the adsorbent contains 0.05-0.8 wt%, preferably 0.1-0.5 wt% of adsorbed water.

**16.** The method according to any one of claims 8 to 15, wherein the operating conditions of the adsorption in step 1) and of the desorption in step 2) each independently include:

a temperature of 100-190 °C, preferably 110-180 °C, more preferably 120-160 °C; and/or
a pressure of 0.6-1.6 MPa, preferably 0.8-1.0 MPa.

**17.** The method according to any one of claims 8 to 16, wherein step 1) and step 2) are performed by using a simulated moving bed, wherein the simulated moving bed comprises a plurality of adsorption bed layers loaded with adsorbents, each bed layer is provided with corresponding material inlet and outlet pipelines, the materials entering and exiting the simulated moving bed divide the adsorption bed layers therein into a desorption zone, a purification zone, an adsorption zone and an isolation zone, wherein the adsorption bed layers between the desorbent injection position and the extract liquid discharging position is the desorption zone, the adsorption bed layers between the extract liquid discharging position and the feedstock injection position is the purification zone, the adsorption bed layers between the feedstock injection position and the raffinate discharging position are the adsorption zone, and the adsorption bed layers between the raffinate discharging position and the desorbent injection position are the isolation zone,

preferably, the ratio of the number of bed layers in the adsorption zone, purification zone, desorption zone and isolation zone in the simulated moving bed is $25\pm10\%$: $38\pm15\%$: $25\pm5\%$: $12\pm4\%$,
further preferably, the mass flow rate ratio of the desorbent to the feedstock entering the simulated moving bed is 0.01-6.0, preferably 0.01-5.5, more preferably 0.01-5.0,
more preferably, the flow rate of the feedstock entering the simulated moving bed relative to the unit mass of the adsorbent is in the range of 0.1-8 kg/(h·kg adsorbent), preferably 0.15-8 kg/(h·kg adsorbent), more preferably 0.17-8 kg/(h· kg adsorbent),
particularly preferably, one cycle period of the simulated moving bed is 12-70 minutes, preferably 20-40 minutes.

**18.** An adsorption-desorption agents kit, comprising a solid adsorbent and a liquid desorbent, wherein the solid adsorbent comprises at least 90 wt% of Y-type molecular sieve as an active component, the Y-type molecular sieve having a silicon oxide/aluminum oxide molar ratio of 4.0-6.0, preferably 4.3-5.7, and the liquid desorbent comprises or consists of 20-100 wt% of an alkylbenzene compound of the following general formula (I) and 0-80 wt% of a C5-C14 saturated aliphatic hydrocarbon,

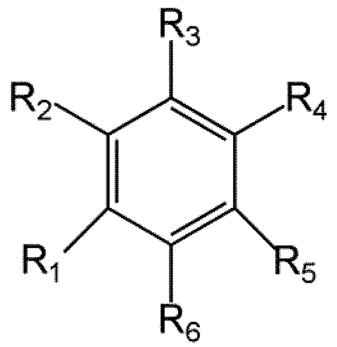

(I),

wherein:

$R_1$, $R_2$ and $R_3$, which may be the same or different, are independently selected from $C_{1-4}$ chain alkyl groups, preferably, at least one of $R_1$, $R_2$ and $R_3$ is methyl, more preferably at least two of them are methyl, and further preferably all of them are methyl;

$R_4$, $R_5$ and $R_6$, which may be the same or different, are independently selected from hydrogen, $C_{1-4}$ saturated hydrocarbon group, $C_{1-4}$ alkoxy group and halogen, preferably, at least one of $R_4$, $R_5$ and $R_6$ is hydrogen or $C_{1-4}$ saturated hydrocarbon group, for example, $R_4$ or $R_6$ is $C_{1-4}$ saturated hydrocarbon group, more preferably, at least two of them are independently hydrogen or $C_{1-4}$ saturated hydrocarbon group, for example, $R_4$ and $R_6$ are both $C_{1-4}$ saturated hydrocarbon group, further preferably, three of them are independently hydrogen or $C_{1-4}$ saturated hydrocarbon group, and particularly preferably, all three are hydrogen, wherein the alkyl group is preferably methyl.

**19.** The kit according to claim 18, wherein, based on the total amount of the liquid desorbent, the desorbent comprises or consists of 30-95 wt%, preferably 30-80 wt%, more preferably 30-50 wt% of the alkylbenzene compound, and 5-70 wt%, preferably 20-70 wt%, more preferably 50-70 wt% of alkanes selected from C5-C14 normal alkanes, preferably selected from C6-C10 normal alkanes.

**20.** The kit according to claim 18 or 19, wherein in formula (I), at least two of $R_1$, $R_2$ and $R_3$ are methyl, $R_4$, $R_5$ and $R_6$ are independently hydrogen or $C_{1-4}$ saturated hydrocarbon groups, and at least one of them is hydrogen; preferably, at least two of $R_4$, $R_5$ and $R_6$ are hydrogen, and the $C_{1-4}$ saturated hydrocarbon group is methyl;
more preferably, the alkylbenzene compound is selected from 1,2,3-trimethylbenzene, 1,2,3,4-tetramethylbenzene, 1,2,3,4,5-pentamethylbenzene and 3-ethyl-o-xylene, preferably 1,2,3-trimethylbenzene or 1,2,3,4-tetramethylbenzene.

**21.** The kit according to any one of claims 18 to 20, wherein the adsorbent comprises one or more, preferably one or two, of groups IA, IIA and IB metal ions as charge-balancing cations, wherein the group IA metal ions are preferably selected from $Li^+$ and $Na^+$, the group IIA metal ions are preferably selected from $Mg^{2+}$, $Sr^{2+}$ and $Ba^{2+}$, and the group IB metal ions are preferably $Ag^+$, preferably, the total amount of groups IA, IIA and IB metals in the adsorbent is 10-45%, calculated as metal oxide and based on the total amount of the adsorbent,

preferably, the crystal size of the Y-type molecular sieve in the solid adsorbent is 0.5-2.0 $\mu$m, preferably 0.8-1.2 $\mu$m,
further preferably, the adsorbent contains 0.05-2 wt%, preferably 0.05-1 wt%, more preferably 0.05-0.8 wt%, further preferably 0.1-0.5 wt% of adsorbed water,
more preferably, the adsorbent contains at least 90 wt% of NaY-type molecular sieve as an active component, and is preferably treated with metal ion exchange to achieve a metal ion exchange degree of 78.0-99.9%.

**22.** The kit according to claim 21, wherein the liquid desorbent comprises 30-50 wt% of the alkylbenzene compound and 50-70 wt% of an alkane selected from C5-C14 normal alkanes, based on the total amount of the liquid desorbent; and the charge-balancing cations of the solid adsorbent are selected from $Na^+$, $Sr^{2+}$, $Ba^{2+}$ and $Ag^+$, and the adsorbent contains 0.05-0.8 wt%, preferably 0.1-0.5 wt% of adsorbed water.

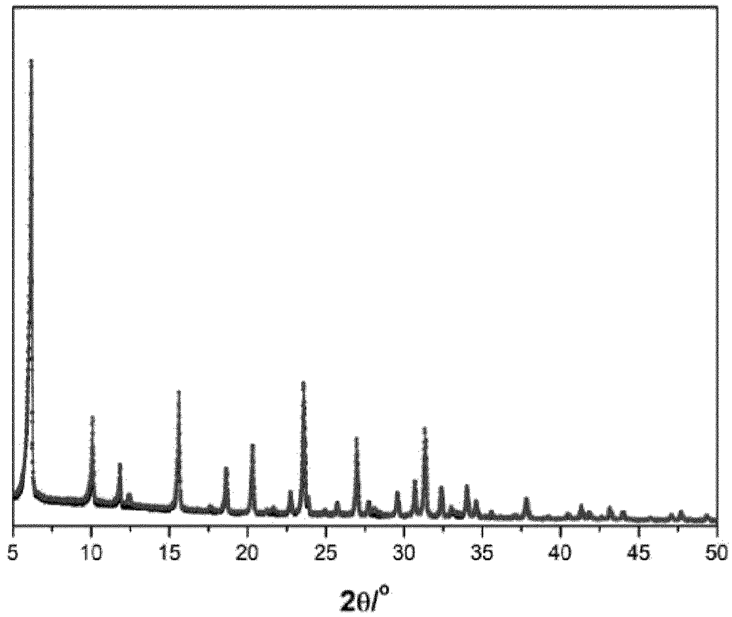
5
10
15
20
25
30
35
40
45
50
2θ/°

FIG. 1

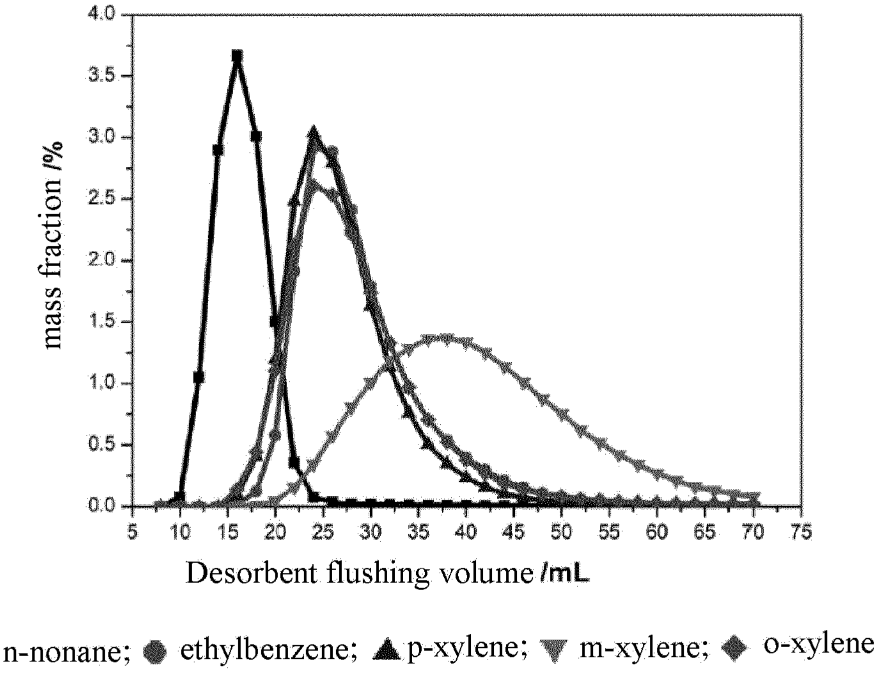
4.0
3.5
3.0
2.5
2.0
1.5
1.0
0.5
0.0
mass fraction /%
5
10
15
20
25
30
35
40
45
50
55
60
65
70
75
Desorbent flushing volume /mL
■ n-nonane; ● ethylbenzene; ▲ p-xylene; ▼ m-xylene; ◆ o-xylene

FIG. 2

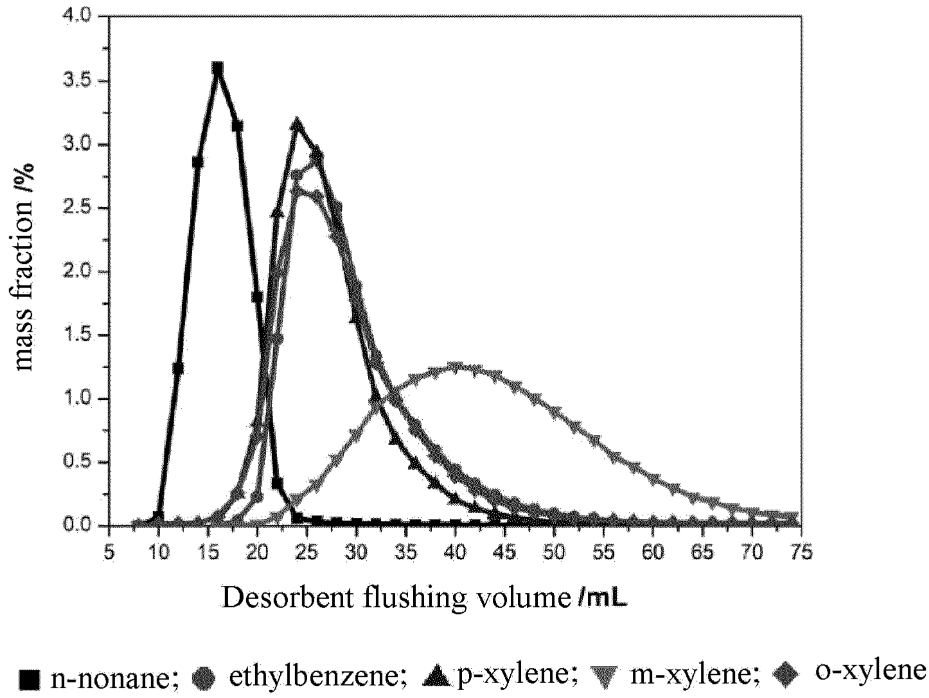
mass fraction /%
4.0
3.5
3.0
2.5
2.0
1.5
1.0
0.5
0.0
5 10 15 20 25 30 35 40 45 50 55 60 65 70 75
Desorbent flushing volume /mL
■ n-nonane; ● ethylbenzene; ▲ p-xylene; ▼ m-xylene; ◆ o-xylene

FIG. 3

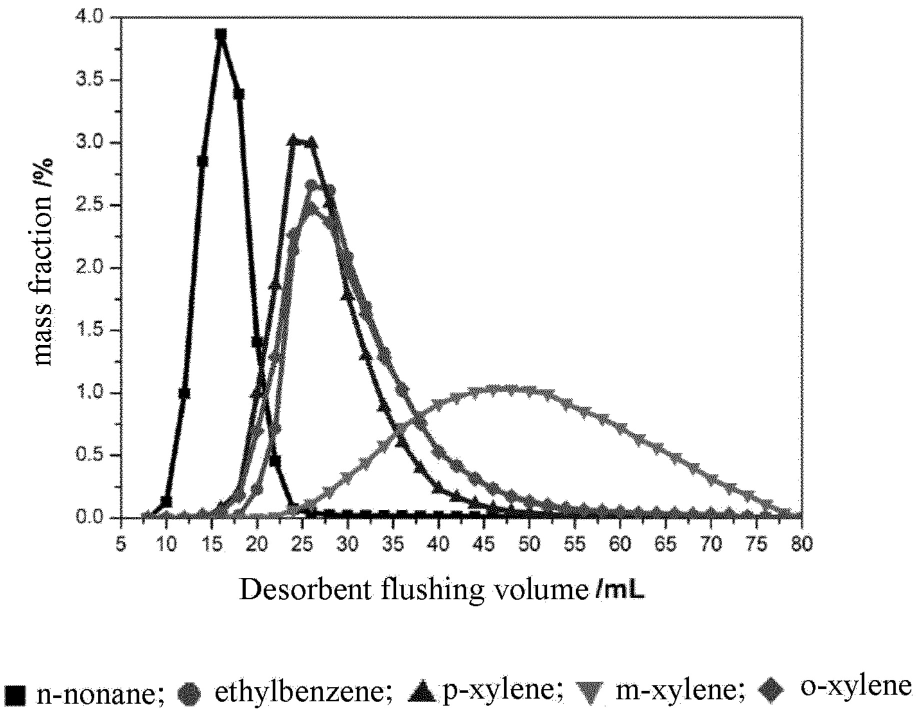
mass fraction /%
4.0
3.5
3.0
2.5
2.0
1.5
1.0
0.5
0.0
5 10 15 20 25 30 35 40 45 50 55 60 65 70 75 80
Desorbent flushing volume /mL
■ n-nonane; ● ethylbenzene; ▲ p-xylene; ▼ m-xylene; ◆ o-xylene

FIG. 4

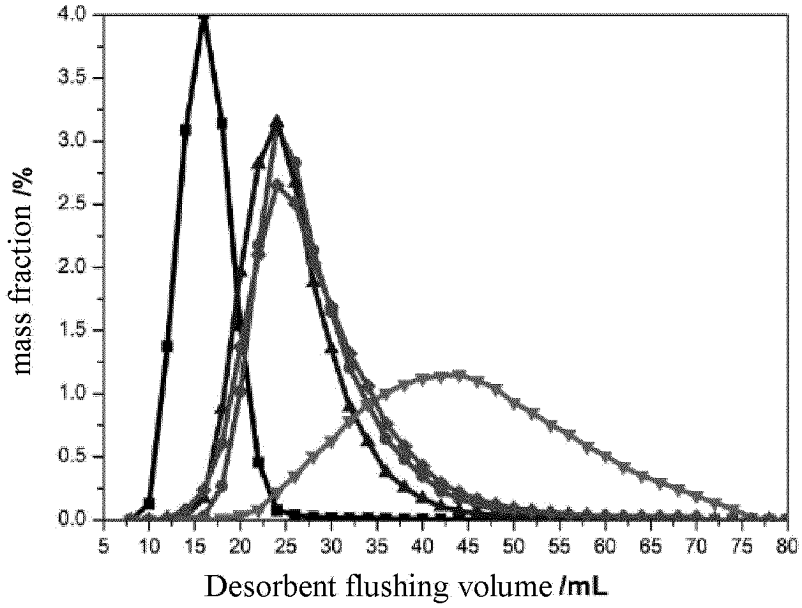


■ n-nonane; ● ethylbenzene; ▲ p-xylene; ▼ m-xylene; ◆ o-xylene

FIG. 5

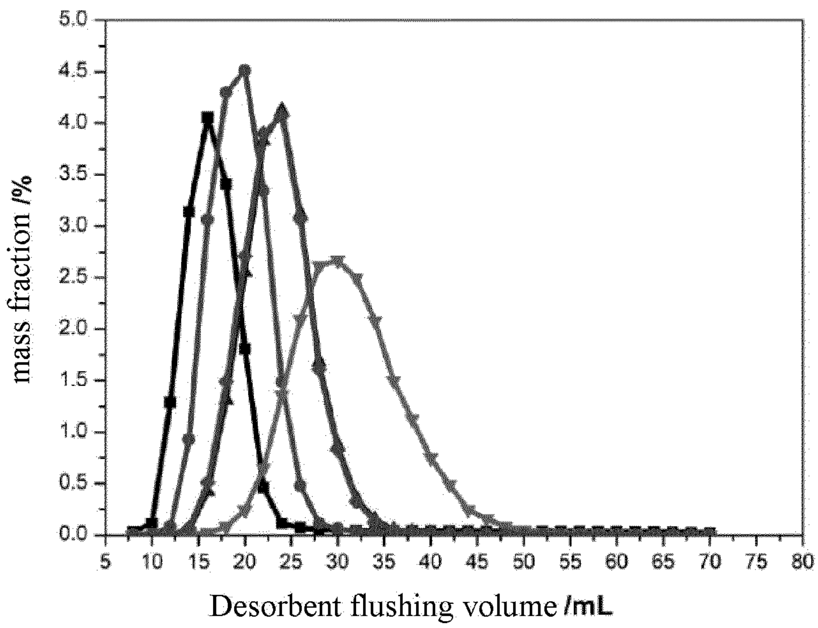


■ n-nonane; ● ethylbenzene; ▲ p-xylene; ▼ m-xylene; ◆ o-xylene

FIG. 6

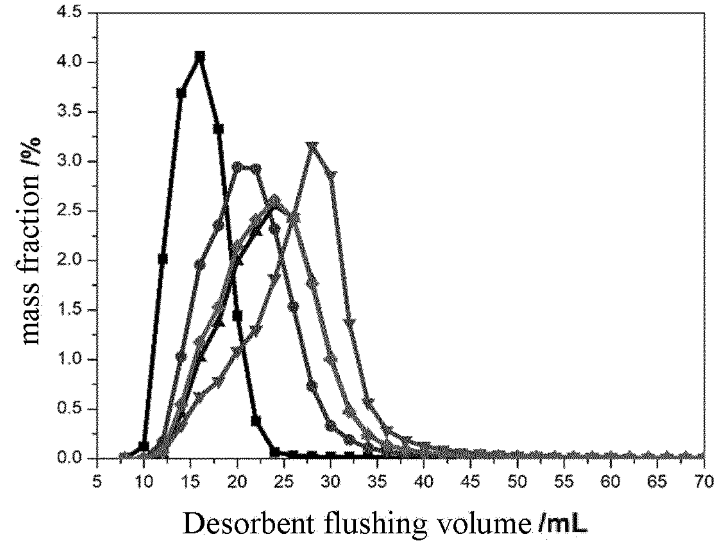


■ n-nonane; ● ethylbenzene; ▲ p-xylene; ▼ m-xylene; ◆ o-xylene

FIG. 7

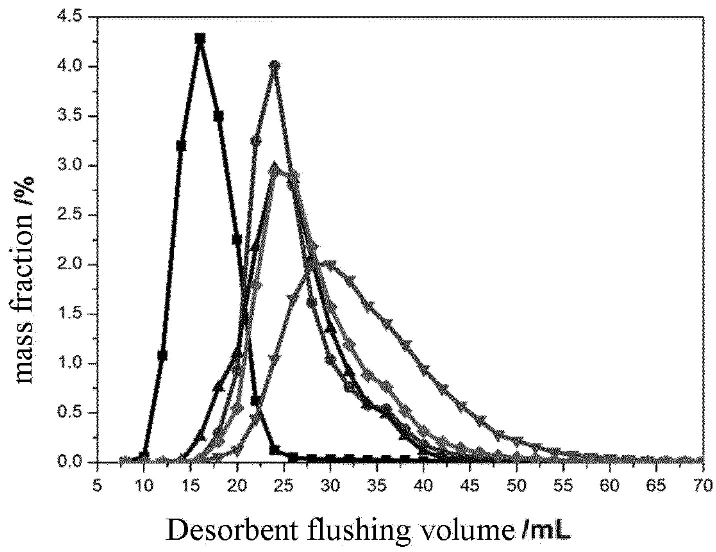


■ n-nonane; ● ethylbenzene; ▲ p-xylene; ▼ m-xylene; ◆ o-xylene

FIG. 8

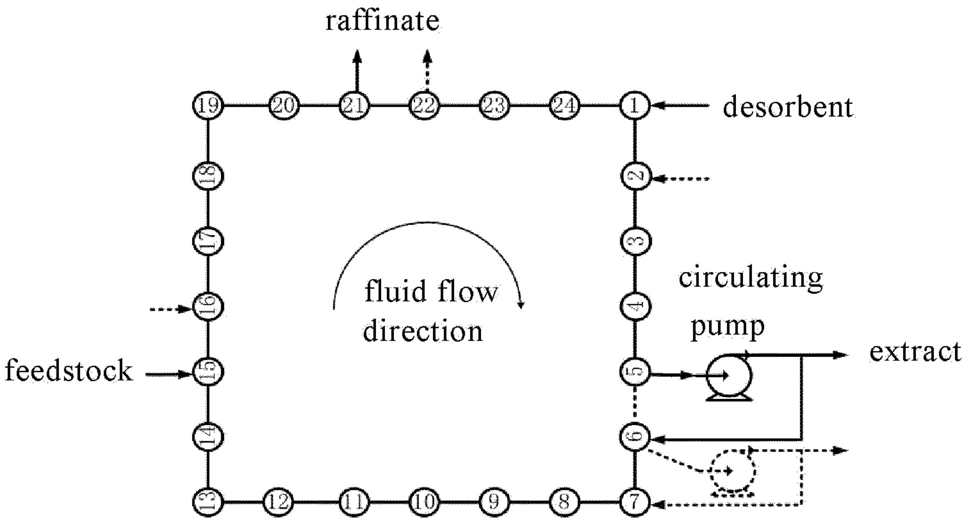
raffinate
19
20
21
22
23
24
1
desorbent
2
18
17
3
circulating
pump
fluid flow
direction
4
16
feedstock
15
5
extract
14
6
13
12
11
10
9
8
7

FIG. 9

**INTERNATIONAL SEARCH REPORT**

International application No. **PCT/CN2023/106757**

**A. CLASSIFICATION OF SUBJECT MATTER**

C07C7/13(2006.01)i；C07C15/08(2006.01)i

According to International Patent Classification (IPC) or to both national classification and IPC

**B. FIELDS SEARCHED**

Minimum documentation searched (classification system followed by classification symbols)

IPC:C07C

Documentation searched other than minimum documentation to the extent that such documents are included in the fields searched

Electronic data base consulted during the international search (name of data base and, where practicable, search terms used)

CNABS, DWPI, CNKI, Caplus(STN), Registry(STN): 二甲苯, 三甲苯, 饱和, 庚烷, 辛烷, 壬烷, 癸烷, 银, 吸附, 解吸, 脱吸, xylene, mesitylene, saturated, heptane, octane, nonane, decane, silver, adsorption, desorption

**C. DOCUMENTS CONSIDERED TO BE RELEVANT**

| Category* | Citation of document, with indication, where appropriate, of the relevant passages | Relevant to claim No. |
|---|---|---|
| X | JP S52934 B1 (TEIJIN LTD.) 11 January 1977 (1977-01-11) claim 1, and description, columns 6-8 and 10-12 | 1-4, 6, 8-10, 12-14, 16-18, 20, 21 |
| X | JP H069438 A (CHIYODA CHEMICAL ENGINEERING & CONSTRUCTION COMPANY LIMITED) 18 January 1994 (1994-01-18) claim 1, and description, paragraphs 12-14, 17-19, and 23-24 | 1-4, 6, 8-10, 12-14, 16-18, 20, 21 |
| Y | JP S52934 B1 (TEIJIN LTD.) 11 January 1977 (1977-01-11) claim 1, and description, columns 6-8 and 10-12 | 1-22 |
| Y | JP H069438 A (CHIYODA CHEMICAL ENGINEERING & CONSTRUCTION COMPANY LIMITED) 18 January 1994 (1994-01-18) claim 1, and description, paragraphs 12-14, 17-19, and 23-24 | 1-22 |
| Y | CN 101745364 A (CHINA PETROCHEMICAL CO., LTD. et al.) 23 June 2010 (2010-06-23) claim 1, and description, paragraph 38 | 1-22 |
| A | JP H08217700 A (CHIYODA CHEMICAL ENGINEERING & CONSTRUCTION COMPANY LIMITED) 27 August 1996 (1996-08-27) entire document | 1-22 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 September 2023** | **10 October 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/106757**

**C. DOCUMENTS CONSIDERED TO BE RELEVANT**

| Category* | Citation of document, with indication, where appropriate, of the relevant passages | Relevant to claim No. |
|---|---|---|
| A | US 4368347 A (SISAS S.P.A.) 11 January 1983 (1983-01-11)<br>entire document | 1-22 |

Form PCT/ISA/210 (second sheet) (July 2022)

**INTERNATIONAL SEARCH REPORT**
**Information on patent family members**

International application No.
**PCT/CN2023/106757**

| Patent document cited in search report | Publication date (day/month/year) | Patent family member(s) | Publication date (day/month/year) |
|---|---|---|---|
| JP S52934 B1 | 11 January 1977 | None | |
| JP H069438 A | 18 January 1994 | None | |
| CN 101745364 A | 23 June 2010 | None | |
| JP H08217700 A | 27 August 1996 | None | |
| US 4368347 A | 11 January 1983 | EP 0045953 A1 | 17 February 1982 |
| | | EP 0045953 B1 | 16 January 1985 |
| | | KR 830006147 A | 17 September 1983 |
| | | KR 840001305 B1 | 11 September 1984 |
| | | DE 3168309 D1 | 28 February 1985 |
| | | IT 8024044 D0 | 07 August 1980 |
| | | IT 1132538 B | 02 July 1986 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- CN 101745364 A
- CN 101772478 A
- CN 1939883 A
- CN 1379007 A
- US 5900523 A

### Non-patent literature cited in the description

- *CHEMICAL ABSTRACTS*, 526-73-8
- *CHEMICAL ABSTRACTS*, 108-88-3